# EUROPEAN PATENT APPLICATION

(11) **EP 4 831 284 A1**
(43) Date of publication of application: **28.10.2026**
(21) Application number: 24917417.8
(22) Date of filing: 30.09.2024
(51) Int. Cl.: C12N 9/18, C12P 7/44, C08J 11/10

(54) **NOVEL PET DEGRADING ENZYME AND USE THEREOF**

(30) Priority: 11.01.2024 KR 20240004885; 11.01.2024 KR 20240004886; 11.01.2024 KR 20240004887
(71) Applicant: Kyungpook National University Industry-Academic Cooperation Foundation, Daegu 41566 (KR); CJ Cheiljedang Corporation, Seoul 04560 (KR)
(72) Inventor: KIM, Kyung-Jin, Daegu 42038 (KR); JANG, Jaewon, Seoul 04560 (KR); HONG, Hwaseok, Seoul 04560 (KR); RYU, Ae Jin, Seoul 04560 (KR); KIM, Hyung Joon, Seoul 04560 (KR)
(74) Representative: Meissner Bolte Partnerschaft mbB
(86) International application number: PCT/KR2024/014898
(87) International publication number: WO 2025/150658

(57) **Abstract**

The present disclosure relates to a novel PET degrading enzyme and use thereof.

## Description

### [Technical Field]

The present disclosure relates to a polypeptide having PET degradation activity and a use thereof.

### [Background Art]

Over 400 million tons of plastics are newly produced every year, and as environmental issues regarding waste plastics emerge, efforts are being made to reduce production thereof through regulations on single-use products and use of plastic substitutes. However, the use of plastics is actually increasing every year. PET, which accounts for less than 10% of all plastics, is newly produced at about 360 million tons every year, and is considered as a plastic with the shortest life cycle because it is mainly used for single-use products. Recycling of waste plastics comprises mechanical recycling, pyrolysis, chemical recycling, *etc*., and each method is commercialized or entering the final research stage for commercialization. Each technology may be a solution to the waste plastic issue, but no existing method can be considered perfect due to issues such as quality deterioration by downcycling, carbon neutrality, resource depletion, the impact on eutrophication of seawater and freshwater, *etc*.

In order to solve environmental problems caused by waste plastics, such as microplastics, greenhouse gas emissions, and resource depletion, *etc*., a series of research findings are being published for degrading PET, a representative type of plastic, by using a biological technology that utilizes enzymes.

### [Prior Art Documents]

(Patent Document 1) EP 3,909,947 A2

### [Disclosure]

### [Technical Problem]

The present disclosure relates to a polypeptide having PET degradation activity and a use thereof.

### [Technical Solution]

One object of the present disclosure is to provide a variant polypeptide having PET degradation activity.

Another object of the present disclosure is to provide a composition comprising the polypeptide.

Still another object of the present disclosure is to provide a polynucleotide encoding the polypeptide.

Still another object of the present disclosure is to provide a host cell comprising: the polypeptide; a polynucleotide encoding the same; a nucleic acid construct comprising the polynucleotide; and/or a vector comprising the nucleotide or the nucleic acid construct.

Still another object of the present disclosure is to provide a method for preparing a variant polypeptide having PET degradation activity.

Still another object of the present disclosure is to provide a method for degrading polyester, comprising treating polyester with a polypeptide having PET degradation activity; a host cell expressing the polypeptide; and/or a composition comprising the polypeptide.

Still another object of the present disclosure is to provide a method for preparing bis-2-hydroxyethyl terephthalate (BHET), (mono(2-hydroxymethyl) terephthalate (MHET), terephthalic acid (TPA), and/or ethylene glycol (EG), comprising contacting polyester with a polypeptide having PET degradation activity; a host cell expressing the polypeptide; and/or a composition comprising the polypeptide.

Still another object of the present disclosure is to provide a method for producing polyester, comprising synthesizing polyester using BHET, MHET, TPA, and/or EG prepared according to the method.

Still another object of the present disclosure is to provide a use of a polypeptide having PET degradation activity, a host cell expressing the polypeptide, and/or a composition comprising the polypeptide, for degrading PET.

Still another object of the present disclosure is to provide a use of a polypeptide having PET degradation activity; a host cell expressing the polypeptide; and/or a composition comprising the polypeptide for reaction with polyester in preparing bis-2-hydroxyethyl terephthalate (BHET), (mono(2-hydroxymethyl) terephthalate (MHET), terephthalic acid (TPA), and/or ethylene glycol (EG).

### [Advantageous Effects]

The variant polypeptide having PET degradation activity of the present disclosure can be usefully employed in various industries.

### [Brief Description of the Drawings]

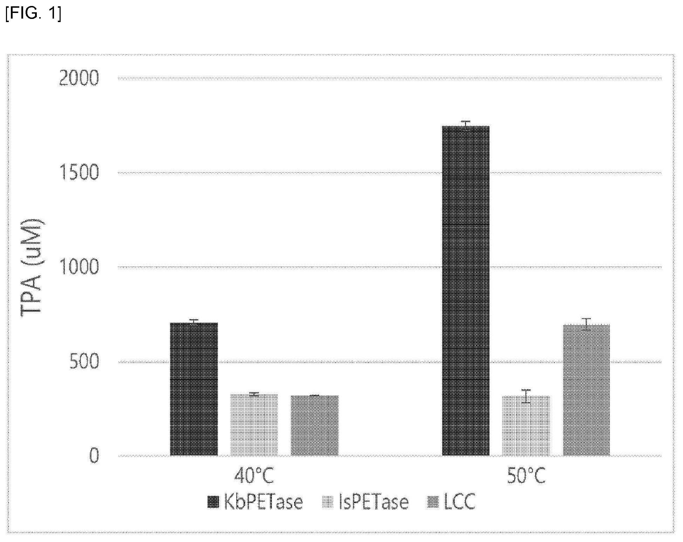

FIG. 1 confirms the PET degradation activity of KbPETase, LCC, and IsPETase at 40°C and 50°C.

### [Detailed Description of Preferred Embodiments]

One aspect of the present disclosure provides a variant polypeptide having PET degradation activity.

In one embodiment, the variant polypeptide is i) a polypeptide having sequence identity of at least 70% and less than 100% to SEQ ID NO: 1; and/or
ii) the variant polypeptide is a polypeptide encoded by a polynucleotide having sequence identity of at least 70% and less than 100% to a sequence encoding a mature polypeptide of SEQ ID NO: 1; and/or
iii) the variant polypeptide is a polypeptide encoded by (a) a mature polypeptide coding sequence of SEQ ID NO: 1, (b) a cDNA thereof, or (c) a polynucleotide that hybridizes to the full-length complement of (a) or (b) under low stringency conditions, medium stringency conditions, medium-high stringency conditions, high stringency conditions, or very high stringency conditions; and/or
iv) the variant polypeptide is a functional fragment of a polypeptide of i) to iii) having PET degradation activity, and
wherein the variant polypeptide comprises any one modification selected from the following at one or more positions selected from positions 95, 119, 184, 239, 279, 173, 197, 236, 281, 127, 131, and 190:
   amino acid deletion, amino acid insertion, substitution with another amino acid, disulfide bond formation, and/or a combination thereof,
   wherein the position numbers correspond to the positions in the polypeptide of SEQ ID NO: 1.

In any one embodiment of the foregoing embodiment, prior to modification, the amino acid at position 95 may be threonine (T); the amino acid at position 119 may be threonine (T); the amino acid at position 184 may be valine (V); the amino acid at position 239 may be aspartate (D); the amino acid at position 279 may be alanine (A); the amino acid at position 173 may be alanine (A); the amino acid at position 197 may be lysine (K); the amino acid at position 236 may be alanine (A); the amino acid at position 281 may be serine (S); the amino acid at position 127 may be glutamine (Q); the amino acid at position 131 may be glutamate (E); and/or the amino acid at position 190 may be aspartate (D).

In any one embodiment of the foregoing embodiments, the variant polypeptide may comprise one or more of the following substitutions:
substitution of the amino acid corresponding to position 95 with arginine, histidine, or lysine;
substitution of the amino acid corresponding to position 119 with asparagine, glutamine, arginine, histidine, lysine, aspartate, or glutamate;
substitution of the amino acid corresponding to position 184 with isoleucine;
substitution of the amino acid corresponding to position 239 with serine or threonine;
substitution of the amino acid corresponding to position 279 with serine;
substitution of the amino acid corresponding to position 173 with cysteine, isoleucine, leucine, or valine;
substitution of the amino acid corresponding to position 197 with cysteine, alanine, isoleucine, leucine, or valine;
substitution of the amino acid corresponding to position 236 with cysteine, leucine, or serine;
substitution of the amino acid corresponding to position 281 with cysteine, alanine, or leucine;
substitution of the amino acid corresponding to position 127 with serine;
substitution of the amino acid corresponding to position 131 with glutamine, asparagine, or cysteine; and
substitution of the amino acid corresponding to position 190 with histidine, arginine, lysine, or glutamate,
wherein the position numbers correspond to the positions in the polypeptide of SEQ ID NO: 1.

In any one embodiment of the foregoing embodiments, the variant polypeptide may comprise one or more substitutions selected from:
one or more substitutions selected from T95R, D239S, A279S, T119N, and V184I;
A173C/K197C;
A236C/S281C; and
one or more substitutions selected from Q127S, E131Q, and D190H,
wherein the position numbers correspond to the positions in the polypeptide of SEQ ID NO: 1.

In any one embodiment of the foregoing embodiments, the variant polypeptide may comprise one or more substitutions selected from:
T95R/D239S/A279S/T119N/V184I;
A173C/K197C;
A236C/S281C; and
Q127S/E131Q/D190H,
wherein the position numbers correspond to the positions in the polypeptide of SEQ ID NO: 1.

In any one embodiment of the foregoing embodiments, the variant polypeptide may be one in which:
the amino acid at position 95 is substituted with arginine; the amino acid at position 239 is substituted with serine; the amino acid at position 279 is substituted with serine; the amino acid at position 119 is substituted with asparagine; the amino acid at position 184 is substituted with isoleucine; the amino acid at position 173 is substituted with cysteine; the amino acid at position 197 is substituted with cysteine; the amino acid at position 236 is substituted with cysteine; and the amino acid at position 281 is substituted with cysteine,
wherein the position numbers correspond to the positions in the polypeptide of SEQ ID NO: 1.

In any one embodiment of the foregoing embodiments, the variant polypeptide may be one in which:
the amino acid at position 173 is substituted with cysteine; the amino acid at position 197 is substituted with cysteine; the amino acid at position 236 is substituted with cysteine; the amino acid at position 281 is substituted with cysteine; the amino acid at position 127 is substituted with serine; the amino acid at position 131 is substituted with glutamine; and the amino acid at position 190 is substituted with histidine,
wherein the position numbers correspond to the positions in the polypeptide of SEQ ID NO: 1.

In any one embodiment of the foregoing embodiments, the variant polypeptide may be one in which:
the amino acid at position 95 is substituted with arginine; the amino acid at position 239 is substituted with serine; the amino acid at position 279 is substituted with serine; the amino acid at position 119 is substituted with asparagine; the amino acid at position 184 is substituted with isoleucine; the amino acid at position 173 is substituted with cysteine; the amino acid at position 197 is substituted with cysteine; the amino acid at position 236 is substituted with cysteine; the amino acid at position 281 is substituted with cysteine; the amino acid at position 127 is substituted with serine; the amino acid at position 131 is substituted with glutamine; and the amino acid at position 190 is substituted with histidine,
wherein the position numbers correspond to the positions in the polypeptide of SEQ ID NO: 1.

In any one embodiment of the foregoing embodiments, the variant polypeptide forms a disulfide bond between a cysteine pair selected from the following:
cysteine at position 173 and cysteine at position 197; and/or
cysteine at position 236 and cysteine at position 281,
wherein the position numbers correspond to the positions in the polypeptide of SEQ ID NO: 1.

In any one embodiment of the foregoing embodiments, the variant polypeptide may have one or more of modified properties the following i) to vii) relative to a polypeptide consisting of the amino acid sequence of SEQ ID NO: 1:
i) increase or decrease in enzymatic activity;
ii) increase or decrease in a specific activity;
iii) increase or decrease in pH stability;
iv) increase or decrease in storage stability;
v) increase or decrease in acid tolerance;
vi) increase or decrease in thermal tolerance or thermal stability; and
vii) alteration of substrate specificity.

Another aspect of the present disclosure provides a composition comprising the variant polypeptide having PET degradation activity.

In any one embodiment of the foregoing embodiments, the composition may be a composition for degrading PET.

Still another aspect of the present disclosure provides a polynucleotide encoding the polypeptide.

Still another aspect of the present disclosure provides a nucleic acid construct comprising the polynucleotide.

Still another aspect of the present disclosure provides a vector comprising the polynucleotide or the nucleic acid construct.

Still another aspect of the present disclosure provides a host cell comprising the variant polypeptide, the polynucleotide, the nucleic acid construct, and/or the vector.

Still another aspect of the present disclosure provides a method for preparing a variant polypeptide, comprising culturing the host cell; and recovering the variant polypeptide expressed in the culturing.

Still another aspect of the present disclosure provides a method for degrading polyester, comprising treating polyester with the variant polypeptide or a polypeptide of SEQ ID NO: 1 or having at least 70% sequence identity thereto; a host cell expressing the polypeptide; and/or a composition comprising the polypeptide.

In any one embodiment of the foregoing embodiments, the polyester may be PET.

In any one embodiment of the foregoing embodiments, the method for degrading polyester may comprise performing a glycolysis reaction by the polypeptide having PET degradation activity.

In any one embodiment of the foregoing embodiments, the method for degrading polyester may comprise converting the polyester to BHET in the presence of the polypeptide having PET degradation activity and ethylene glycol.

Still another aspect of the present disclosure provides a method for preparing BHET, MHET, TPA, and/or EG, comprising contacting polyester with the variant polypeptide or a polypeptide of SEQ ID NO: 1 or having at least 70% sequence identity thereto; a host cell expressing the polypeptide; and/or a composition comprising the polypeptide.

In any one embodiment of the foregoing embodiments, the polyester may be PET.

In any one embodiment of the foregoing embodiments, the method may further comprise recovering BHET, MHET, TPA, and/or EG prepared by the method.

Still another aspect of the present disclosure provides a method for producing polyester, comprising synthesizing a polyester using the prepared BHET, MHET, TPA, and/or EG.

In any one embodiment of the foregoing embodiments, the polyester may be PET.

Still another aspect of the present disclosure provides a host cell over-expressing the variant polypeptide or a polypeptide of SEQ ID NO: 1 or having at least 70% sequence identity thereto.

Still another aspect of the present disclosure provides a use of the variant polypeptide or a polypeptide of SEQ ID NO: 1 or having at least 70% sequence identity thereto; a host cell expressing the polypeptide; and/or a composition comprising the polypeptide for degrading PET.

Still another aspect of the present disclosure provides use of the variant polypeptide or a polypeptide of SEQ ID NO: 1 or having at least 70% sequence identity thereto; a host cell expressing the polypeptide; and/or a composition comprising the polypeptide for reaction with polyester in preparing bis-2-hydroxyethyl terephthalate (BHET), (mono(2-hydroxymethyl) terephthalate (MHET), terephthalic acid (TPA), and/or ethylene glycol (EG).

### [Modes for Carrying Out the Invention]

The present disclosure will be described in detail with reference to specific embodiments as follows. Meanwhile, each description and embodiment described herein can be applied to other descriptions and embodiments, respectively. That is, all combinations of various elements disclosed herein fall within the scope of the present disclosure. Further, the scope of the present disclosure is not limited by the specific description described below.

Furthermore, those skilled in the art may recognize or identify numerous equivalents to the specific aspects of the present disclosure described herein by using routine experimentation. Moreover, these equivalents are intended to be comprised in the present disclosure.

As used in the specification and appended claims of the present disclosure, the singular articles ("a", "an", and "the") comprise plural referents unless the context clearly indicates otherwise. Further, unless otherwise indicated by the context, singular terms comprise their plural forms, and plural terms comprise their singular forms. As used in the specification and appended claims of the present disclosure, the term "or" may comprise the meaning of "and/or", unless indicated otherwise.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. In one example, the term "about" can refer to a range of -10% to +10% of the numerical value. As another example, the term "about" may refer to a range of -5% to +5% of the given numerical value. However, the term is not limited thereto.

As used herein, terms such as "first, second, third,...", "i), ii), iii),...", or "(a), (b), (c), (d),..." are used to distinguish similar elements and do not imply that the elements are executed continuously or in the listed order. For example, when these terms are used in relation to a method, use, or analytical step, the steps may be performed without any time intervals, simultaneously, or with intervals of several seconds, minutes, hours, days, or months.

As used herein, the term "consisting essentially of" may mean that, in the case where the features of the object claimed herein are not substantially affected by the presence of an unspecified component, the unspecified component may be present.

As used herein, the term "consisting of" means that the total ratio of the specific component(s) is 100%. The components or features recited after the term "consisting of" may be essential or mandatory. In some specific embodiments, in addition to the components or features recited after the term "consisting of", other arbitrary or non-essential components may be excluded.

As used herein, the term "comprising" refers to the presence of the features, steps, or components following the term and does not exclude the presence or addition of one or more other features, steps, or components. As used herein, the components or features following the term "comprising" may be essential or mandatory; however, in certain embodiments, other optional or non-essential components or features may also be comprised.

As used herein, in certain embodiments, the term "comprising" may be modified to refer to "consisting essentially of" or "consisting of".

With respect to amino acid sequences in the present disclosure, even if it is disclosed as a polypeptide "comprising" an amino acid sequence described by a specific sequence number, a polypeptide "consisting of" an amino acid sequence described by a specific sequence number, or a polypeptide or protein "having" an amino acid sequence described by a specific sequence number, it is apparent that a protein having an amino acid sequence in which a portion of the sequence is deleted, modified, substituted, conservatively substituted or added can be used in the present disclosure if it has the same or corresponding activity as the polypeptide consisting of the amino acid sequence of the corresponding sequence number. For example, it may be a case where the N-terminus and/or C-terminus of the amino acid sequence is added with a sequence that does not alter the function of the protein, a naturally occurring mutation, a silent mutation thereof, or a conservative substitution, but is not limited thereto.

As used herein, the term "protein" or "polypeptide" refers to a polymer or oligomer of consecutive amino acid residues. As used herein, "polypeptide", "protein", and "peptide" may be used interchangeably with "amino acid sequence".

In some cases, an amino acid sequence exhibiting activity may be referred to as an "enzyme". In the present disclosure, unless indicated otherwise, amino acid sequences are described in the N-terminal to C-terminal direction.

As used herein, the term "recombinant" with respect to a cell, or nucleic acid, polypeptide, or vector, means that the cell, nucleic acid, polypeptide, or vector has been modified by the introduction of a heterologous nucleic acid or polypeptide, or the alteration of a native nucleic acid or polypeptide, or that the cell is derived from a cell thus modified. Therefore, for example, recombinant cells may express genes that are not found within the native (non-recombinant) form of the cells, or may express native genes that are otherwise abnormally expressed, under-expressed or not expressed at all.

As used herein, the term "isolated" refers to a substance that exists in a non-naturally occurring environment or does not exist naturally. It comprises at least substantially isolating a substance (sequence, enzyme, or nucleic acid) from a substance that is naturally associated and found in nature, for example, at least one other component having a sequence, enzyme, or nucleic acid.

For example, an isolated sequence, enzyme, or nucleic acid provided in the present disclosure may be provided in a form that is substantially free of one or more contaminants.

Examples of isolated substances comprise: i) any non-naturally occurring substance; ii) any substance (*e.g.*, enzyme, variant, nucleic acid, protein, peptide, or cofactor) from which one, or more, or all naturally-occurring components associated in nature have been removed; iii) any substance that has been artificially modified from a substance found in nature; or iv) any substance that has been modified to alter the amount of that substance relative to other components associated in nature (*e.g.*, increase in copy number of a gene encoding a specific substance; modification of a promoter naturally linked to a gene encoding a specific substance into a highly active promoter, *etc.*), but are not limited thereto.

As used herein, the term "wild-type" refers to a naturally-occurring state without artificial modification. When the term "wild-type" is used in reference to a polypeptide, it refers to a naturally occurring polypeptide which does not have artificial mutations (substitutions, insertions, deletions, *etc.*) at one or more amino acid positions. Similarly, when the term "wild-type" is used in reference to a polynucleotide, it refers to a polynucleotide lacking artificial modifications (substitutions, insertions, deletions) of one or more nucleotides. However, polynucleotides encoding wild-type polypeptides are not limited to native polynucleotides and comprise sequences encoding any wild-type polypeptides.

As used herein, the term "parent sequence" or "backbone" refers to a reference sequence in which modification is introduced to create a modified polypeptide. That is, the parent sequence may be a starting sequence into which modifications such as substitutions, additions, and/or deletions are to be introduced. The parent sequence may be naturally-occurring or wild-type, or a variant in which one or more substitutions, insertions or deletions have occurred in the natural or wild type, or may be an artificially synthesized sequence. When the parent sequence is an amino acid sequence exhibiting activity, *i.e.*, an amino acid sequence of an enzyme, it may be referred to as a parent enzyme.

As used herein, the term "reference sequence" refers to a sequence used to determine the position of amino acids within any amino acid sequence. By aligning any amino acid sequence with a reference sequence, the position of an amino acid within the sequence that corresponds to a specific position in the reference sequence can be determined.

As used herein, in reference to amino acid or nucleic acid sequences, the term "fragment" refers to a portion of a parent sequence. For example, it may be a polypeptide in the form in which one or more amino acids are removed from the C- or N-terminus of the parent sequence.

As used herein, the "fragment" of an enzyme may refer to a "functional fragment". The term "functional fragment" may also be referred to as an active fragment, and refers to a polypeptide that is a portion of a parent enzyme and has enzymatic activity of the parent enzyme. For example, the functional fragment of an enzyme may comprise a catalytic site of the enzyme.

A fragment of the enzyme may comprise a portion of the full length of the parent enzyme. For example, it may comprise amino acids of at least about 20%, 30%, 40%, 50%, 60%, 70%, 80%, 90%, 95%, 98%, 99% or more, and less than 100% of the full length of the parent enzyme, but is not limited thereto.

In the present disclosure, "modifying" means changing or altering. It may be altering from a naturally occurring state. In one example, an enzyme may be altered in a manner that causes it to differ from a parent sequence or a reference sequence.

In the present disclosure, a modified enzyme may be an enzyme that does not exist in nature by itself, *i.e.*, a non-naturally occurring enzyme.

As used herein, the term "modified" refers, for example, to something that has been altered from its naturally occurring state. The modified enzyme of the present disclosure comprises non-naturally occurring enzymes or naturally occurring variants. For example, the modified enzyme of the present disclosure is a modified enzyme that has not been found in nature. For example, the modified enzyme of the present disclosure may be an enzyme that does not occur spontaneously, but is not limited thereto.

As used herein, the term "modification", when used with respect to an amino acid/nucleic acid sequence, may comprise: substitution of an amino acid/nucleic acid residue of a parent sequence for a different amino acid/nucleic acid residue at one or more positions in the amino acid sequence; deletion of an amino acid/nucleic acid residue (or a series of amino acid/nucleic acid residues) of a parent sequence at one or more positions; insertion of an amino acid/nucleic acid residue (or a series of amino acid/nucleic acid residues) of a parent sequence at one or more positions; truncation of the amino acid sequence at the N-terminus and/or C-terminus or of the nucleic acid sequence at the 5' and/or 3' end; and any combination thereof.

As used herein, the "variant" or "modified polypeptide" of an enzyme refers to a protein having one or more amino acids different from the parent enzyme by conservative substitution and/or modification. The "variant" or "modified polypeptide" may be used interchangeably. The variant or modified polypeptide may be non-naturally occurring, but is not limited thereto.

The variant differs from the sequence of the parent enzyme by one or more modifications, for example, substitutions, deletions, and/or insertions of amino acids.

Such variants may generally be identified by modifying one or more amino acids from the parent enzyme and evaluating the properties of the modified protein. That is, the ability of a variant may be enhanced, unchanged, or reduced relative to the parent enzyme.

Additionally, certain variants may comprise a variant polypeptide in which one or more regions, such as an N-terminal leader sequence or transmembrane domain, have been removed.

Other variants may comprise a variant in which a portion has been removed from the N- and/or C-terminus of the mature protein.

The term "variant" or "variant polypeptide" may be used interchangeably with terms comprising modification, modified protein, mutant, mutein, divergent, and variant, and is not limited as long as the terms are used to indicate mutation.

The variant may also comprise deletion or addition of amino acids that have minimal influence on the properties and secondary structure of a polypeptide. For example, a polypeptide may be conjugated with a signal (or leader) sequence at the N-terminus of a protein involved in the translocation of proteins co-translationally or post-translationally. Further, the polypeptide may also be conjugated with another sequence or linker to allow for identification, purification, or synthesis of the polypeptide.

As used herein, the term "conservative substitution" refers to the substitution of an amino acid with another amino acid having similar structural and/or chemical properties. Such an amino acid substitution may generally occur based on similarity in polarity, charge, solubility, hydrophobicity, hydrophilicity, and/or amphipathic nature of the residues.

Throughout the entire specification of the present disclosure, the conventional one-letter and three-letter codes for naturally occurring amino acids are used. Additionally, the amino acids mentioned herein are abbreviated according to the IUPAC-IUB nomenclature as follows:

| | |
|---|---|
| alanine Ala, A | arginine Arg, R |
| asparagine Asn, N | aspartate Asp, D |
| cysteine Cys, C | glutamate Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

Meanwhile, any amino acid may be described as Xaa or X.

In addition to naturally occurring amino acids, commonly accepted three-letter codes may also be used for other amino acids such as 2-aminoisobutyric acid (Aib), N-methylglycine (Sar), and α-methyl-glutamate.

Amino acids can generally be classified based on similarity in polarity, charge, solubility, hydrophobicity, hydrophilicity, and/or amphipathic nature of the residues. Accordingly, amino acid substitution may generally occur based on similarity in polarity, charge, solubility, hydrophobicity, hydrophilicity, and/or amphipathic nature of the residues.

For example, among the amino acids having an electrically charged side chain (electrically charged amino acids), positively charged (basic) amino acids comprise arginine, lysine, and histidine, and negatively charged (acidic) amino acids comprise glutamate and aspartate. Further, among the amino acids having an uncharged side chain (uncharged amino acids), nonpolar amino acids comprise glycine, alanine, valine, leucine, isoleucine, methionine, phenylalanine, tryptophan, and proline, and polar or hydrophilic amino acids comprise serine, threonine, cysteine, tyrosine, asparagine, and glutamine. Among the nonpolar amino acids, aromatic amino acids comprise phenylalanine, tryptophan, and tyrosine.

As used herein, the term "gene" means a polynucleotide that encodes a polypeptide and a polynucleotide comprising the upstream and downstream regions of the coding region. In certain embodiments, a gene may have a sequence (intron) inserted between each coding region (exon).

As used herein, the term "homology" or "identity" refers to the degree of relevance between two given amino acid sequences or nucleotide sequences, and may be expressed as a percentage. The terms "homology" and "identity" may often be used interchangeably.

The sequence homology or identity of conserved polynucleotides or polypeptides may be determined by a standard alignment algorithm, and default gap penalties established by the program used may be applied. Substantially, homologous or identical sequences may generally hybridize under moderately or highly stringent conditions to the full-length sequence or to at least about 50%, 60%, 70%, 80%, or 90% or more of the full-length. It is apparent that hybridization also comprises hybridization with polynucleotides containing codons that reflect common codon usage or codon degeneracy in the polynucleotides.

Whether any two polynucleotide or polypeptide sequences have homology, similarity, or identity may be determined by, for example, a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (the GCG program package (Devereux, J. et al., Nucleic Acids Research 12:387 (1984)), BLASTP, BLASTN, and FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073) are comprised). For example, homology, similarity, or identity may be determined using BLAST from the National Center for Biotechnology Information (NCBI) or ClustalW, but is not limited thereto.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. Briefly, the GAP program may be defined as the value obtained by dividing the number of similarly aligned symbols (*i.e.*, nucleotides or amino acids) by the total number of symbols in the shorter of the two sequences. Default parameters for the GAP program may comprise: (1) a unitary matrix (containing a value of 1 for identities and 0 for non-identities), and the weighted comparison matrix (or EDNAFULL (EMBOSS version of NCBI NUC4.4) substitution matrix) of Gribskov et al. (1986) Nucl Acids Res. 14:6745 as disclosed in Schwartz and Dayhoff, eds., Atlas Of Protein Sequence And Structure, National Biomedical Research Foundation (1979), pp. 353-358; (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity may be identified by comparing the sequences in a Southern hybridization experiment under stringent conditions as defined. Appropriate hybridization conditions defined are within the skill of the art, and may be determined by a method well known to a person skilled in the art (*e.g.*, J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York), but is not limited thereto.

As used herein, the term "mature polypeptide" refers to a polypeptide in a form without a signal sequence or propeptide sequence. A mature protein/polypeptide/peptide may be a functional form of a protein/polypeptide/peptide. A mature polypeptide may be in a final form after translation or post-translational modification. Examples of post-translational modification comprise N-terminal or C-terminal modifications, glycosylation, phosphorylation, removal of a leader sequence, *etc*., but are not limited thereto.

As used herein, the term "nucleic acid construct" refers to a single- or doublestranded nucleic acid molecule that comprises one or more regulatory sequences and is artificially synthesized, engineered to comprise a specific sequence in a manner that does not exist in nature, or isolated from nature.

As used herein, the term "expression" comprises any step involved in the production of a polypeptide, for example, transcription, post-transcriptional modification, translation, post-translational modification, and secretion, but is not limited thereto.

As used herein, the term "expression vector" refers to a linear or circular nucleic acid molecule comprising a coding sequence and an operably linked regulatory sequence for expression.

As used herein, the term "operably linked" refers to a configuration in which a regulatory sequence is positioned at an appropriate position to regulate the expression of a coding sequence. Thus, the term "operably linked" comprises attaching or linking a regulatory region of a functional domain having a known or desired activity such as a promoter, a stop codon, a signal sequence, or an enhancer region, with a target (gene or polypeptide) so as to regulate the expression, secretion, or function of the target in accordance with the known or desired activity.

As used herein, the term "cDNA" refers to a DNA sequence that can be produced by reverse transcription from a mature, spliced mRNA molecule obtainable from a eukaryotic or prokaryotic cell. A cDNA sequence lacks intron sequences that may be present in the corresponding genomic DNA. The initial primary RNA transcript is a precursor to mRNA that undergoes a series of steps comprising splicing to become mature spliced mRNA.

As used herein, the term "regulatory sequence" refers to a polynucleotide sequence necessary for the expression of a coding sequence. Each regulatory sequence may be native (derived from the same origin) or foreign (derived from a different gene) to the coding sequence. Examples of the regulatory sequence may comprise a leader sequence, a polyadenylation sequence, a propeptide sequence, a promoter, a signal peptide sequence, an operator sequence, a sequence encoding a ribosome-binding domain, and a sequence regulating the termination of transcription and translation. The minimum unit of the regulatory sequence may comprise a promoter and a sequence for terminating transcription and translation.

In order to describe the variant provided in the present disclosure, the following nomenclature is used.

In the present disclosure, referring to a specific position in an amino acid sequence may comprise referring to an amino acid present or substituted at that position. Referring to an amino acid at a specific position can be described in various ways. For example, "position 003" can be described as "position 3", "amino acid 3", or "the third amino acid". Additionally, when the amino acid at position 3 is serine (S), for example, it may be described as "S3" or "Ser3".

Amino acid substitution can be expressed by describing the amino acid before substitution, the position, and the substituted amino acid in sequence. The amino acids may be expressed using the conventional one-letter and three-letter codes. For example, when alanine at position 8 of a specific sequence is substituted with valine, it may be described as "A8V" or "Ala8Val".

Any amino acid at a specific position may be referred to as "X". For example, X6 refers to any amino acid at position 6. Additionally, when the substituted amino acid is expressed as X, it means that it is substituted with an amino acid different from the amino acid before substitution. For example, "V6X" indicates that V at position 6 is substituted with any amino acid other than V.

Different alterations can be expressed by simultaneously describing several types of amino acids using symbols such as ",". For example, the amino acid (D) at position 12 being substituted with S or K can be written as D12S,K.

Multiple mutations can be described using "/". For example, T95R/D239S means that threonine, the amino acid at position 95, is replaced with arginine, and aspartate, the amino acid at position 239, is replaced with serine.

As used herein, the term "corresponding to" refers to an amino acid residue at the position recited in a protein or peptide, or an amino acid residue that is similar, identical, or homologous to the residue recited in a protein or peptide. Identification of the amino acid at the corresponding position may be determining a specific amino acid in a sequence that references a particular sequence. As used herein, the term "corresponding region" refers to a similar or corresponding position in a related protein or a reference protein.

In the present disclosure, SEQ ID NO: 1 can be used as a reference sequence to determine the position of amino acids in any amino acid sequence.

That is, SEQ ID NO: 1 disclosed herein can be used to determine the corresponding amino acid residues in any polypeptide having PET degradation activity. Unless indicated otherwise in the present disclosure, residues of a specific amino acid sequence are numbered relative to SEQ ID NO: 1.

For example, based on the alignment of any amino acid sequence with SEQ ID NO: 1, each amino acid residue in the amino acid sequence can be numbered with reference to the position number of the amino acid residue corresponding to the amino acid residue of SEQ ID NO: 1. For example, a sequence alignment algorithm as described herein can identify the position of an amino acid or a position where modification such as substitution, insertion, or deletion occurs compared to a query sequence (also referred to as the "reference sequence").

For such alignment, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000), Trends Genet. 16: 276-277), and the like can be used, but are not limited thereto.

Additionally, multiple sequence alignment can be used to identify corresponding amino acid residues in other PETases. Examples of multiple sequence alignment programs known in the art comprise multiple sequence comparison by log-expectation (MUSCLE; version 3.5 or higher; Edgar, 2004, Nucleic Acids Research 32: 1792-1797), MAFFT (version 6.857 or higher; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), EMBOSS EMMA using ClustalW (1.83 or higher; Thompson et al., 1994, Nucleic Acids Research 22 : 4673-4680), and the like, and the basic parameters of each of the programs may be used, but are not limited thereto.

In addition, if the relationship between the enzymes branched from the mature polypeptide of SEQ ID NO: 1 cannot be detected by conventional sequence-based comparison, other pairwise sequence comparison algorithms may be used (Lindahl and Elofsson, 2000, J. Mol. Biol. 295: 613-615). Higher sensitivity can be achieved in sequence-based searches by using search programs that utilize probabilistic representations of polypeptide families (profiles) to search databases. For example, the PSI-BLAST program can generate a profile through an iterative database search process and detect remote homologs (Atschul et al., 1997, Nucleic Acids Res. 25: 3389-3402). When the family or superfamily of a polypeptide has one or more representatives in protein structure databases, significantly higher sensitivity can be achieved. Programs such as GenTHREADER (Jones, 1999, J. Mol. Biol. 287: 797-815; McGuffin and Jones, 2003, Bioinformatics 19: 874-881) use information from various sources, such as PSI-BLAST, secondary structure prediction, structural alignment profiles, and solvation potentials, as input to neural networks that predict structural folding for query sequences. Similarly, the method of Gough et al., 2000, J. Mol. Biol. 313: 903-919 can be used to align sequences of unknown structure with superfamily models existing in the SCOP database. These alignments may be sequentially used to generate homology models for the peptides, and such models can be assessed for accuracy using a variety of tools developed for that purpose.

For proteins of known structure, several tools and resources may be used for retrieving and generating structural alignments. For example, the SCOP superfamilies of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (CE) (Shindyalov and Bourne, 1998, Protein Engineering 11: 739-747), and implementation of these algorithms can additionally be utilized to query structure databases with a structure of interest in order to discover possible structural homologs (Holm and Park, 2000, Bioinformatics 16: 566-567).

The above methods are illustrative examples and are not limited thereto.

Hereinafter, the specific embodiments of the present disclosure will be described in more detail.

In the present disclosure, "polypeptide having PET degradation activity" or "PET-degrading enzyme (PETase)" refers to a polypeptide having depolymerization activity of polyethylene terephthalate (PET), and may also comprise polypeptides having depolymerization activity of low molecular weight polymers, for example, bis-2-hydroxyethyl terephthalate (BHET), obtained by depolymerizing PET. The term "depolymerization" refers to a process in which a polymer or at least one polymer in the plastic material is depolymerized into smaller molecules, such as monomers and/or oligomers.

In the present disclosure, PET degradation activity can be measured and evaluated by using methods known in the art, comprising embodiments described in the present disclosure. For example, the amount of BHET, MHET, or TPA produced can be measured and evaluated.

As used herein, the term "parent PETase" refers to a PET-degrading enzyme that is modified to produce the variant or variant polypeptide of the present disclosure. Specifically, the parent PETase, parent enzyme, or parent sequence may be a naturally occurring or wild-type polypeptide or a mature polypeptide thereof, and may comprise a variant or functional fragment thereof. However, it is not limited thereto as long as it is a polypeptide that has PETase activity and is capable of serving as a parent of a variant.

The parent PETase provided in the present disclosure may be the polypeptide of SEQ ID NO: 1, but is not limited thereto. Further, the parent PETase may be a polypeptide having a sequence identity of about 60%, 70%, 75%, 77%, 80%, 85%, 90%, 95%, 96%, 97%, 98%, or 99% or more to the polypeptide of SEQ ID NO: 1 as long as it exhibits PET degradation activity, and a polypeptide may be comprised in the scope of the parent PETase without limitation if it has the same or equivalent activity as the polypeptide consisting of the amino acid sequence of SEQ ID NO: 1.

The parent PETase of the variant provided in the present disclosure may be derived from a microorganism of the genus *Kutzneria*. Specifically, it may be derived from *Kutzneria buriramensis*.

Meanwhile, the above-described microorganism is an example of a microorganism from which the parent PETase provided in the present disclosure may be derived, and comprises microorganisms derived from taxonomically homologous microorganisms regardless of the name of the microorganism.

The above-described microorganism can be obtained from known microorganism deposit institutions such as ATCC, DSMZ, CBS, NRRL, KCTC, and KCCM.

In the present disclosure, a sequence "derived from" a specific microorganism is not limited to those naturally produced or producible in the microorganism, but also comprises sequences encoded by genes produced and isolated from microorganisms comprising the gene.

For example, PETase derived from a microorganism of the genus *Kutzneria* comprises not only enzymes having PETase activity naturally produced in a microorganism of the genus *Kutzneria,* but also those produced from a source of a microorganism of the genus *Kutzneria*. It also comprises those produced in other host cells through genetic modification known in the art (*e.g.*, transformation with a sequence encoding the enzyme).

In addition, the present disclosure newly identified that the polypeptide of SEQ ID NO: 1 has PET degradation activity, and prepared a variant polypeptide with modified or enhanced properties by introducing a mutation to the parent sequence SEQ ID NO: 1. Accordingly, one aspect of the present disclosure provides a use of a polypeptide having the same or corresponding activity as the polypeptide consisting of the amino acid sequence of SEQ ID NO: 1 as a PET-degrading enzyme. For the polypeptide having the same or corresponding activity as the polypeptide consisting of the amino acid sequence of SEQ ID NO: 1, the description of the parent PET-degrading enzyme of the variant provided in the present disclosure may be applied.

### Variant of Parent Tase

As used herein, the "variant polypeptide having PET degradation activity" may be a variant of a parent PETase.

As used herein, the term "variant of PET-degrading enzyme" or "variant of PET-degrading enzyme" refers to a protein having PET degradation activity in which one or more amino acids differ from the amino acid sequence of the parent PETase.

The above "variant polypeptide having PET degradation activity", "PETase variant", and "variant of PET-degrading enzyme" may be used interchangeably.

The variant polypeptide provided in the present disclosure may have PET degradation activity while comprising modifications of one or more amino acids in the sequence of a parent PETase. The modifications may be amino acid deletion, insertion, substitution with another amino acid, disulfide bond formation, and/or a combination thereof. Specifically, the modification may be amino acid substitution and/or disulfide bond formation, and more specifically, a combination thereof.

Additionally, i) the variant may have a sequence identity of at least 70% and less than 100% to SEQ ID NO: 1; and/or ii) the variant may be a polypeptide encoded by a polynucleotide having a sequence identity of at least 70% and less than 100% to a sequence encoding the mature polypeptide of SEQ ID NO: 1; and/or iii) the variant may be a polypeptide encoded by (a) a mature polypeptide coding sequence of SEQ ID NO: 1, (b) a cDNA thereof, or (c) a polynucleotide that hybridizes to the full-length complement of (a) or (b) under low stringency conditions, medium stringency conditions, medium-high stringency conditions, high stringency conditions, or very high stringency conditions; and/or iv) the variant may be a functional fragment of i), ii), or iii) having PET degradation activity.

Specifically, the variant provided in the present disclosure may have PET degradation activity and comprise one or more amino acid modifications in the sequence of a parent PETase, thereby exhibiting one or more altered functions or properties compared to the parent PETase.

In one embodiment, the variant provided in the present disclosure may have PETase activity and comprise one or more amino acid modifications in the sequence of a parent PETase, thereby exhibiting one or more altered functions or properties compared to the parent PETase, and may have one or more conservative substitutions.

The variant provided in the present disclosure is a variant of a parent PETase and may be a polypeptide having PET degradation activity.

In one embodiment, the variant provided in the present disclosure may comprise modification at one or more selected positions among the amino acids corresponding to positions 95, 119, 184, 239, 279, 173, 197, 236, 281, 127, 131, and 190 of SEQ ID NO: 1.

As used herein, the position numbers correspond to the positions in the polypeptide of SEQ ID NO: 1, and the term "corresponding" is as described above.

In any one embodiment of the foregoing embodiments, the variant provided in the present disclosure may comprise modification of amino acids corresponding to one or more of T95, T119, V184, D239, A279, A173, K197, A236, S281, Q127, E131, and D190 of SEQ ID NO: 1.

In any one embodiment of the foregoing embodiments, prior to modification provided in the present disclosure, the amino acid at position 95 may be threonine (T); the amino acid at position 119 may be threonine (T); the amino acid at position 184 may be valine (V); the amino acid at position 239 may be aspartate (D); the amino acid at position 279 may be alanine (A); the amino acid at position 173 may be alanine (A); the amino acid at position 197 may be lysine (K); the amino acid at position 236 may be alanine (A); the amino acid at position 281 may be serine (S); the amino acid at position 127 may be glutamine (Q); the amino acid at position 131 may be glutamate (E); and/or the amino acid at position 190 may be aspartate (D).

In one embodiment of the foregoing embodiments, the variant polypeptide provided in the present disclosure may comprise substitution of the amino acid at a position corresponding to position 95 of SEQ ID NO: 1 with G, A, V, L, I, M, F, W, P, S, C, Y, N, Q, D, E, K, R, or H, and specifically may comprise substitution of the amino acid with R, H, or K.

In one embodiment of the foregoing embodiments, the variant polypeptide provided in the present disclosure may comprise substitution of the amino acid at a position corresponding to position 119 of SEQ ID NO: 1 with G, A, V, L, I, M, F, W, P, S, C, Y, N, Q, D, E, K, R, or H, and specifically may comprise substitution of the amino acid with N, Q, R, H, K, D, or E.

In one embodiment of the foregoing embodiments, the variant polypeptide provided in the present disclosure may comprise substitution of the amino acid at a position corresponding to position 184 of SEQ ID NO: 1 with G, A, L, I, M, F, W, P, S, T, C, Y, N, Q, D, E, K, R, or H, and specifically may comprise substitution of the amino acid with I.

In one embodiment of the foregoing embodiments, the variant polypeptide provided in the present disclosure may comprise substitution of the amino acid at a position corresponding to position 239 of SEQ ID NO: 1 with G, A, V, L, I, M, F, W, P, S, T, C, Y, N, Q, E, K, R, or H, and specifically may comprise substitution of the amino acid with S or T.

In one embodiment of the foregoing embodiments, the variant polypeptide provided in the present disclosure may comprise substitution of the amino acid at a position corresponding to position 279 of SEQ ID NO: 1 with G, V, L, I, M, F, W, P, S, T, C, Y, N, Q, D, E, K, R, or H, and specifically may comprise substitution of the amino acid with S.

In one embodiment of the foregoing embodiments, the variant polypeptide provided in the present disclosure may comprise substitution of the amino acid at a position corresponding to position 173 of SEQ ID NO: 1 with G, V, L, I, M, F, W, P, S, T, C, Y, N, Q, D, E, K, R, or H, and specifically may comprise substitution of the amino acid with C, I, L, or V.

In one embodiment of the foregoing embodiments, the variant polypeptide provided in the present disclosure may comprise substitution of the amino acid at a position corresponding to position 197 of SEQ ID NO: 1 with G, A, V, L, I, M, F, W, P, S, T, C, Y, N, Q, D, E, R, or H, and specifically may comprise substitution of the amino acid with C, A, I, L, or V.

In one embodiment of the foregoing embodiments, the variant polypeptide provided in the present disclosure may comprise substitution of the amino acid at a position corresponding to position 236 of SEQ ID NO: 1 with G, V, L, I, M, F, W, P, S, T, C, Y, N, Q, D, E, K, R, or H, and specifically may comprise substitution of the amino acid with C, L, or S.

In one embodiment of the foregoing embodiments, the variant polypeptide provided in the present disclosure may comprise substitution of the amino acid at a position corresponding to position 281 of SEQ ID NO: 1 with G, A, V, L, I, M, F, W, P, T, C, Y, N, Q, D, E, K, R, or H, and specifically may comprise substitution of the amino acid with C, A, or L.

In one embodiment of the foregoing embodiments, the variant polypeptide provided in the present disclosure may comprise substitution of the amino acid at a position corresponding to position 127 of SEQ ID NO: 1 with G, A, V, L, I, M, F, W, P, S, T, C, Y, N, D, E, K, R, or H, and specifically may comprise substitution of the amino acid with S.

In one embodiment of the foregoing embodiments, the variant polypeptide provided in the present disclosure may comprise substitution of the amino acid at a position corresponding to position 131 of SEQ ID NO: 1 with G, A, V, L, I, M, F, W, P, S, T, C, Y, N, Q, D, K, R, or H, and specifically may comprise substitution of the amino acid with Q, N, or C.

In one embodiment of the foregoing embodiments, the variant polypeptide provided in the present disclosure may comprise substitution of the amino acid at a position corresponding to position 190 of SEQ ID NO: 1 with G, A, V, L, I, M, F, W, P, S, T, C, Y, N, Q, E, K, R, or H, and specifically may comprise substitution of the amino acid with H, R, K, or E.

In one embodiment of the foregoing embodiments, the variant provided in the present disclosure may comprise one or more of the following substitutions:
substitution of the amino acid corresponding to position 95 with arginine, histidine, or lysine;
substitution of the amino acid corresponding to position 119 with asparagine, glutamine, arginine, histidine, lysine, aspartate, or glutamate;
substitution of the amino acid corresponding to position 184 with isoleucine;
substitution of the amino acid corresponding to position 239 with serine or threonine;
substitution of the amino acid corresponding to position 279 with serine;
substitution of the amino acid corresponding to position 173 with cysteine, isoleucine, leucine, or valine;
substitution of the amino acid corresponding to position 197 with cysteine, alanine, isoleucine, leucine, or valine;
substitution of the amino acid corresponding to position 236 with cysteine, leucine, or serine;
substitution of the amino acid corresponding to position 281 with cysteine, alanine, or leucine;
substitution of the amino acid corresponding to position 127 with serine;
substitution of the amino acid corresponding to position 131 with glutamine, asparagine, or cysteine; and
substitution of the amino acid corresponding to position 190 with histidine, arginine, lysine, or glutamate,
wherein the position numbers correspond to the positions in the polypeptide of SEQ ID NO: 1.

In one embodiment of the foregoing embodiments, the variant provided in the present disclosure may comprise one or more of the following substitutions:
T95R,H,K;
T119N,Q,R,H,K,D,E;
V184I;
D239S,T;
A279S;
A173C, I, L, V;
K197C, A, I, L, V;
A236C, L, S;
S281C, A, L;
Q127S;
E131Q, N, C; and
D190H, R, K, E;
wherein the position numbers correspond to the positions in the polypeptide of SEQ ID NO: 1.

In one embodiment of the foregoing embodiments, the variant provided in the present disclosure may comprise substitution of the amino acids at positions 173 and 297 of SEQ ID NO: 1 with cysteine, and may form a disulfide bridge (disulfide bond) between the substituted amino acids.

In one embodiment of the foregoing embodiments, the variant provided in the present disclosure may comprise substitution of the amino acids at positions 236 and 281 of SEQ ID NO: 1 with cysteine, and may form a disulfide bridge between the substituted amino acids.

In one embodiment of the foregoing embodiments, the variant provided in the present disclosure may comprise: one or more substitutions selected from A173C/K197C, and A236C/S281C; and one or more substitutions selected from Q127S, E131Q,N,C, and D190H,R,K,E; and/or one or more substitutions selected from T95R,H,K, T119N,Q,R,H,K,D,E, V184I, D239S,T, and A279S.

In one embodiment of the foregoing embodiments, the variant provided in the present disclosure may comprise one or more substitutions selected from:
A173I/K197I;
A173L/K197L;
A173V/K197V;
A236L/S281L;
Q127S/E131N; and
Q127S/E131C.

In one embodiment of the foregoing embodiments, the variant provided in the present disclosure may comprise one or more of the following substitutions:
one or more substitutions selected from T95R, D239S, A279S, T119N, and V184I;
A173C/K197C;
A236C/S281C; and
one or more substitutions selected from Q127S, E131Q, and D190H,
wherein the position numbers correspond to the positions in the polypeptide of SEQ ID NO: 1.

In one embodiment of the foregoing embodiments, the variant provided in the present disclosure may comprise one or more of the following substitutions:
T95R/D239S/A279S/T119N/V184I;
A173C/K197C;
A236C/S281C; and
Q127S/E131Q/D190H;
wherein the position numbers correspond to the positions in the polypeptide of SEQ ID NO: 1.

In one embodiment,
a variant comprising T95R substitution in SEQ ID NO: 1 may be represented by SEQ ID NO: 3,
a variant comprising D239S substitution in SEQ ID NO: 1 may be represented by SEQ ID NO: 4,
a variant comprising A279S substitution in SEQ ID NO: 1 may be represented by SEQ ID NO: 5,
a variant comprising T119N substitution in SEQ ID NO: 1 may be represented by SEQ ID NO: 6,
a variant comprising V184I substitution in SEQ ID NO: 1 may be represented by SEQ ID NO: 7,
a variant comprising T95R/D239S substitution in SEQ ID NO: 1 may be represented by SEQ ID NO: 8,
a variant comprising T95R/A279S substitution in SEQ ID NO: 1 may be represented by SEQ ID NO: 9,
a variant comprising T95R/T119N substitution in SEQ ID NO: 1 may be represented by SEQ ID NO: 10,
a variant comprising T95R/V184I substitution in SEQ ID NO: 1 may be represented by SEQ ID NO: 11,
a variant comprising D239S/A279S substitution in SEQ ID NO: 1 may be represented by SEQ ID NO: 12,
a variant comprising D239S/T119N substitution in SEQ ID NO: 1 may be represented by SEQ ID NO: 13,
a variant comprising D239S/V184I substitution in SEQ ID NO: 1 may be represented by SEQ ID NO: 14,
a variant comprising A279S/T119N substitution in SEQ ID NO: 1 may be represented by SEQ ID NO: 15,
a variant comprising A279S/V184I substitution in SEQ ID NO: 1 may be represented by SEQ ID NO: 16,
a variant comprising T119N/V184I substitution in SEQ ID NO: 1 may be represented by SEQ ID NO: 17,
a variant comprising T95R/D239S/A279S substitution in SEQ ID NO: 1 may be represented by SEQ ID NO: 18,
a variant comprising T95R/D239S/T119N substitution in SEQ ID NO: 1 may be represented by SEQ ID NO: 19,
a variant comprising T95R/D239S/V184I substitution in SEQ ID NO: 1 may be represented by SEQ ID NO: 20,
a variant comprising T95R/A279S/T119N substitution in SEQ ID NO: 1 may be represented by SEQ ID NO: 21,
a variant comprising T95R/A279S/V184I substitution in SEQ ID NO: 1 may be represented by SEQ ID NO: 22,
a variant comprising T95R/T119N/V184I substitution in SEQ ID NO: 1 may be represented by SEQ ID NO: 23,
a variant comprising D239S/A279S/T119N substitution in SEQ ID NO: 1 may be represented by SEQ ID NO: 24,
a variant comprising D239S/A279S/V184I substitution in SEQ ID NO: 1 may be represented by SEQ ID NO: 25,
a variant comprising D239S/T119N/V184I substitution in SEQ ID NO: 1 may be represented by SEQ ID NO: 26,
a variant comprising A279S/T119N/V184I substitution in SEQ ID NO: 1 may be represented by SEQ ID NO: 27,
a variant comprising T95R/D239S/A279S/T119N substitution in SEQ ID NO: 1 may be represented by SEQ ID NO: 28,
a variant comprising T95R/D239S/A279S/V184I substitution in SEQ ID NO: 1 may be represented by SEQ ID NO: 29,
a variant comprising T95R/D239S/T119N/V184I substitution in SEQ ID NO: 1 may be represented by SEQ ID NO: 30,
a variant comprising T95R/A279S/T119N/V184I substitution in SEQ ID NO: 1 may be represented by SEQ ID NO: 31,
a variant comprising D239S/A279S/T119N/V184I substitution in SEQ ID NO: 1 may be represented by SEQ ID NO: 32,
a variant comprising T95R/D239S/A279S/T119N/V184I substitution in SEQ ID NO: 1 may be represented by SEQ ID NO: 33,
a variant comprising A173C/K197C substitution in SEQ ID NO: 1 may be represented by SEQ ID NO: 34,
a variant comprising A236C/S281C substitution in SEQ ID NO: 1 may be represented by SEQ ID NO: 35,
a variant comprising A173C/K197C/A236C/S281C substitution in SEQ ID NO: 1 may be represented by SEQ ID NO: 36,
a variant comprising E131Q substitution in SEQ ID NO: 1 may be represented by SEQ ID NO: 37,
a variant comprising Q127S/E131Q substitution in SEQ ID NO: 1 may be represented by SEQ ID NO: 38,
a variant comprising D190H substitution in SEQ ID NO: 1 may be represented by SEQ ID NO: 39,
a variant comprising Q127S/E131Q/D190H substitution in SEQ ID NO: 1 may be represented by SEQ ID NO: 40,
a variant comprising T95R/D239S/A279S/T119N/V184I/A173C/K197C/A236C/S281C substitution in SEQ ID NO: 1 may be represented by SEQ ID NO: 41,
a variant comprising A173C/K197C/A236C/S281C/Q127S/E131Q/D190H substitution in SEQ ID NO: 1 may be represented by SEQ ID NO: 42,
a variant comprising T95R/D239S/A279S/T119N/V184I/A173C/K197C/A236C/S281C/Q127S/E131Q/D190H substitution in SEQ ID NO: 1 may be represented by SEQ ID NO: 43,
a variant comprising T95H substitution in SEQ ID NO: 1 may be represented by SEQ ID NO: 44,
a variant comprising T95K substitution in SEQ ID NO: 1 may be represented by SEQ ID NO: 45,
a variant comprising D239T substitution in SEQ ID NO: 1 may be represented by SEQ ID NO: 46,
a variant comprising K197A substitution in SEQ ID NO: 1 may be represented by SEQ ID NO: 47,
a variant comprising A173I/K197I substitution in SEQ ID NO: 1 may be represented by SEQ ID NO: 48,
a variant comprising A173L/K197L substitution in SEQ ID NO: 1 may be represented by SEQ ID NO: 49,
a variant comprising A173V/K197V substitution in SEQ ID NO: 1 may be represented by SEQ ID NO: 50,
a variant comprising T119Q substitution in SEQ ID NO: 1 may be represented by SEQ ID NO: 51,
a variant comprising T119R substitution in SEQ ID NO: 1 may be represented by SEQ ID NO: 52,
a variant comprising T119H substitution in SEQ ID NO: 1 may be represented by SEQ ID NO: 53,
a variant comprising T119K substitution in SEQ ID NO: 1 may be represented by SEQ ID NO: 54,
a variant comprising T119D substitution in SEQ ID NO: 1 may be represented by SEQ ID NO: 55,
a variant comprising T119E substitution in SEQ ID NO: 1 may be represented by SEQ ID NO: 56,
a variant comprising S281A substitution in SEQ ID NO: 1 may be represented by SEQ ID NO: 57,
a variant comprising A236L/S281L substitution in SEQ ID NO: 1 may be represented by SEQ ID NO: 58,
a variant comprising A236S substitution in SEQ ID NO: 1 may be represented by SEQ ID NO: 59,
a variant comprising E131N substitution in SEQ ID NO: 1 may be represented by SEQ ID NO: 60,
a variant comprising D190R substitution in SEQ ID NO: 1 may be represented by SEQ ID NO: 61,
a variant comprising D190K substitution in SEQ ID NO: 1 may be represented by SEQ ID NO: 62,
a variant comprising D190E substitution in SEQ ID NO: 1 may be represented by SEQ ID NO: 63,
a variant comprising Q127S/E131N substitution in SEQ ID NO: 1 may be represented by SEQ ID NO: 64, and
a variant comprising Q127S/E131C substitution in SEQ ID NO: 1 may be represented by SEQ ID NO: 65.

In one embodiment, the variants provided in the present disclosure comprise all possible combinations of the above-described variants.

In one embodiment, the variant polypeptide provided in the present disclosure may have a sequence identity of about 60% or more, for example, 65% or more, 70% or more, 75% or more, 77% or more, 80% or more, 85% or more, 90% or more, 91% or more, 92% or more, 93% or more, 94% or more, 95% or more, 96% or more, 97% or more, 98% or more, or 99% or more, and less than 100% to a parent PETase, a mature polypeptide thereof, or a functional fragment thereof.

In one embodiment, the variant provided in the present disclosure may have a sequence identity of about 60% or more, for example, 65% or more, 70% or more, 75% or more, 77% or more, 80% or more, 85% or more, 90% or more, 91% or more, 92% or more, 93% or more, 94% or more, 95% or more, 96% or more, 97% or more, 98% or more, or 99% or more, and less than 100% to SEQ ID NO: 1.

In one embodiment, the variant provided in the present disclosure may be a polypeptide encoded by a polynucleotide having identity of about 60% or more, for example, 65% or more, 70% or more, 75% or more, 77% or more, 80% or more, 85% or more, 90% or more, 91% or more, 92% or more, 93% or more, 94% or more, 95% or more, 96% or more, 97% or more, 98% or more, or 99% or more, and less than 100% to a sequence encoding the mature polypeptide of SEQ ID NO: 1.

In one embodiment, the variant provided in the present disclosure may have a sequence identity of about 60% or more, for example, 65% or more, 70% or more, 75% or more, 77% or more, 80% or more, 85% or more, 90% or more, 91% or more, 92% or more, 93% or more, 94% or more, 95% or more, 96% or more, 97% or more, 98% or more, or 99% or more, and less than 100% to a functional fragment of SEQ ID NO: 1.

The variant polypeptide provided in the present disclosure may exhibit alterations in one or more selectable or detectable properties or attributes of a polypeptide as compared to other parent PETases, such as wild-type PET-degrading enzymes, parent PETases, and other variant polypeptides having PET degradation activity.

The properties or attributes comprise oxidative stability, substrate specificity, catalytic activity, thermal stability, alkaline stability, pH activity profile, resistance to proteolytic degradation, Km, k_{cat}, k_{cat}/Km ratio, protein folding, immune response induction, ability to bind to a ligand, ability to bind to a receptor, ability to be secreted, ability to be displayed on the surface of a cell, ability to oligomerize, ability to send a signal, ability to stimulate cell proliferation, ability to inhibit cell proliferation, ability to induce apoptosis, ability to be modified by phosphorylation or glycosylation, and/or ability to treat diseases, *etc*., but are not limited thereto.

Specifically, the variant provided in the present disclosure may have one or more of the following altered activities compared to the parent sequence:
i) increase or decrease in enzymatic activity;
ii) increase or decrease in a specific activity;
iii) increase or decrease in pH stability;
iv) increase or decrease in storage stability;
v) increase or decrease in acid tolerance;
vi) increase or decrease in thermal tolerance or thermal stability; and
vii) alteration of substrate specificity.

However, it is not limited thereto.

In another example, the PET-degrading enzyme provided in the present disclosure may have one or more altered activities compared to LCC (GenBank: AEV21261.1) and IsPETase (GenBank: GAP38373.1):
i) increase in enzymatic activity;
ii) increase in a specific activity;
iii) increase in pH stability;
iv) increase in storage stability;
v) increase in acid tolerance;
vi) increase in thermal tolerance; and
vii) alteration of substrate specificity.

However, it is not limited thereto.

In the present disclosure, "enzymatic activity" represents at least one catalytic activity. Specifically, it may be the conversion efficiency of an enzyme, mainly expressed as k _{cat} /Km, but is not limited thereto.

k_{cat} refers to the catalytic constant, which is the rate at which a single enzyme converts a substrate into a product per unit time when the enzyme is completely saturated with the substrate, and is also referred to as the turnover number. Km refers to the substrate concentration at which the reaction rate is at half the maximum value (Vmax).

Examples of the methods of expressing enzymatic activity comprise specific activity (µmol of converted substrate x mg⁻¹ x min⁻¹) or volumetric activity (µmol of converted substrate x mL⁻¹ x min⁻¹).

However, defining enzymatic activity is not limited to the description above and can be defined and evaluated based on known information disclosed in Irwin H. Segel, Enzyme kinetics, John Wiley & Sons, 1979; A. G. Marangoni, Enzyme kinetics, Wiley-Interscience, 2003; A. Fersht, Enzyme structure and mechanisms, John Wiley & Sons, 1981; Structure and Mechanism in Protein Science: A guide to enzyme catalysis and protein folding, Alan Fersht, W. H. Freeman, 1999; Fundamentals of Enzyme Kinetics, Athel Cornish-Bowden, Wiley-Blackwell 2012 and Voet et al., "Biochemie" [Biochemistry], 1992, VCH-Verlag, Chapter 13, pages 331-332 with respect to enzymatic activity, and the like.

In one embodiment, the variant provided in the present disclosure may have increased enzymatic activity by about 100%, about 110%, about 120%, about 130%, about 140%, about 150%, about 160%, about 170%, about 180%, about 190%, or about 200% or more compared to the parent enzyme.

In another embodiment, the variant provided in the present disclosure may have reduced enzymatic activity of about 99%, about 95%, about 90%, about 80%, about 70%, about 60%, about 50%, about 40%, or about 20% or less compared to the parent enzyme.

As used herein, the term "specific activity" refers to the activity of an enzyme per unit weight of protein, and can be expressed as unit/mg. The quantification of protein can be performed using, for example, SDS-PAGE or Bradford assay.

Enzyme stability means that enzymatic activity is retained during storage or reaction time. In order to measure such changes in stability, the initial enzymatic activity can be measured and compared under defined conditions at time zero (100%) and after a certain period of time (x %), and thus, the level at which the enzymatic activity is lost or enzyme stability can be expressed.

Factors that affect enzymatic activity comprise, for example, pH, heat, the presence of other substances (*e.g.*, oxidizing agents, chelating agents), *etc*.

As used herein, the term "pH stability" refers to the ability of a protein to function within a specific pH range. In one embodiment, the variant provided in the present disclosure may be active at about pH 4.0 to about pH 12.0, but is not limited thereto.

If a protein maintains its function within a specific pH range, it can be defined as having "pH stability", and depending on the pH range, it can be defined as having "acid tolerance", "alkali tolerance", *etc*.

As used herein, the term "thermal stability" refers to the ability of a protein to function in a specific temperature range. In one embodiment, the variant provided in the present disclosure may have activity in the range of about 20°C to about 90°C, and specifically may have activity in the range of about 25°C to about 75°C, but is not limited thereto.

As used herein, the term "thermal tolerance" refers to the ability of a protein to function after exposure to a specific temperature, *e.g.*, high heat or cryogenic temperature. For example, proteins with thermal tolerance may not function at non-optimal temperatures, but may regain their function upon returning to optimal temperature conditions.

The increase in stability comprises maintaining high enzymatic activity; and an increased range of pH, temperature, and/or time over which the protein maintains its function, compared to other enzymes, for example, wild-type enzymes, parent enzymes, and/or other variants.

The reduction in stability comprises maintaining low enzymatic activity; and a reduced range of pH, temperature, and/or time over which the protein maintains its function, compared to other enzymes, for example, wild-type enzymes, parent enzymes, and/or other variants.

As used herein, the term "substrate specificity" refers to the ability of an enzyme to identify a substrate and molecules that compete with the substrate. Substrate specificity can be determined by measuring the activity of an enzyme toward different substrates. In one embodiment, the alteration of substrate specificity may be a change toward increased specificity for a substrate that can produce the desired product. In another embodiment, the alteration of substrate specificity may be a change toward reduced specificity for a substrate capable of producing a desired product.

The "polynucleotide" encoding the variant of the present disclosure may comprise the coding sequence of the above-described variant. The polynucleotide may undergo various modifications in the coding region within the scope that does not change the amino acid sequence of the polypeptide, taking into consideration codon degeneracy or the codons preferred in an organism in which the polypeptide is to be expressed.

Additionally, the polynucleotide of the present disclosure may comprise, without limitation, a probe that can be prepared from a known gene sequence, for example, any polynucleotide sequence that hybridizes with a sequence complementary to all or part of the base sequence under stringent conditions to encode the variant of the present disclosure.

The term "stringent conditions" refers to conditions that enable specific hybridization between polynucleotides. These conditions are specifically disclosed in the literature (*e.g.*, J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

For example, the stringent conditions may comprise conditions under which polynucleotides having high homology or identity, *i.e.*, polynucleotides having 40% or more, specifically 90% or more, more specifically 95% or more, 96% or more, 97% or more, or 98% or more, and even more specifically 99% or more homology or identity hybridize with each other, while polynucleotides having homology or identity lower than the above homology or identity do not hybridize with each other; or may comprise ordinary washing conditions of Southern hybridization, *i.e.*, washing once, specifically twice or three times, at a salt concentration and temperature corresponding to 60°C, 1×SSC, 0.1% SDS, specifically 60°C, 0.1×SSC, 0.1% SDS, and more specifically 68°C, 0.1×SSC, 0.1% SDS.

Hybridization requires that two nucleic acids have complementary sequences, although base mismatches are permissible depending on the stringency of the hybridization. The term "complementary" is used to describe a relationship between nucleotide bases that can hybridize with each other. For example, regarding DNA, adenosine is complementary to thymine, and cytosine is complementary to guanine. Therefore, the polynucleotide of the present disclosure may also comprise an isolated nucleic acid fragment complementary to the entire sequence as well as a nucleic acid sequence substantially similar thereto.

Specifically, polynucleotides having homology or identity may be detected using hybridization conditions comprising hybridization at a Tₘ value of 55°C, as well as the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing a polynucleotide depends on the length and degree of complementarity of the polynucleotide, and the variables are well known in the relevant technical field (see J. Sambrook *et al.*, supra, 9.50-9.51, 11.7-11.8).

For example, "high stringency" occurs about 5°C to 10°C below Tm of the probe; "medium stringency" occurs about 10°C to 20°C below Tm of the probe; and "low stringency" may occur about 20°C to 25°C below Tm, but is not limited thereto.

For example, a "low stringency condition" may be prehybridization and hybridization at 42°C in 5X SSPE, 0.3% SDS, sheared and denatured salmon sperm DNA (200 µg/mL) and 25% formamide for 12 to 24 hours according to the Southern blotting standard procedure for probes of at least 100 nucleotides in length. The carrier material can finally be washed 2 to 3 times for 15 minutes each, using 2 X SSC and 0.1% to 0.2% SDS at 50°C.

For example, a "medium stringency condition" may be prehybridization and hybridization at 42°C in 5X SSPE, 0.3% SDS, sheared and denatured salmon sperm DNA (200 µg/mL) and 35% formamide for 12 to 24 hours according to Southern blotting standard procedures for probes of at least 100 nucleotides in length. The carrier material can finally be washed 2 to 3 times for 15 minutes each, using 2 X SSC and 0.1% to 0.2% SDS at 55°C. For example, a "medium-high stringency condition" may be prehybridization and hybridization at 42°C in 5X SSPE, 0.3% SDS, shear and denatured salmon sperm DNA (200 µg/mL) and 35% formamide for 12 to 24 hours according to the Southern blotting standard procedure for probes of at least 100 nucleotides in length. The carrier material can finally be washed 2 to 3 times each for 15 minutes, using 1 to 2 X SSC and 0.1% to 0.2% SDS at 60°C.

For example, a "high stringency condition" may be prehybridization and hybridization at 42°C in 5 X SSPE, 0.3% SDS, sheared and denatured salmon sperm DNA (200 µg/mL) and 35% formamide for 12 to 24 hours according to the Southern blotting standard procedure for probes of at least 100 nucleotides in length. The carrier material can finally be washed 2 to 3 times for 15 minutes each, using 2 X SSC and 0.1% to 0.2% SDS at 65°C.

The "nucleic acid construct" provided in the present disclosure comprises a polynucleotide encoding the variant provided in the present disclosure, that is operably linked to one or more regulatory sequences that direct the expression of a coding sequence in a suitable host cell under conditions suitable for the regulatory sequences.

Polynucleotides can be manipulated in various ways to enable the expression of the variant. Depending on the expression vector, it may be desirable or necessary to manipulate the polynucleotide before inserting the polynucleotide into the vector. Such manipulation may use methods known in the art.

The "vector" provided in the present disclosure refers to a DNA product containing the base sequence of a polynucleotide encoding the variant, which is operably linked to a suitable expression control region (or expression control sequence) so as to enable the expression of the variant in a suitable host. The expression regulatory sequence may comprise a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

The vector that can be used in the present disclosure is not particularly limited, and any vector known in the art may be used. Examples of commonly used vectors comprise plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, and Charon21A, *etc*. may be used; and as a plasmid vector, those based on pBR, pUC, pBluescriptII, pGEM, pTZ, pCL, pET, *etc*. may be used. Specifically, pDZ, pACYC177, pACYC184, pCL, pECCG117, pUC19, pBR322, pMW118, and pCC1BAC vectors may be used.

In an example, a polynucleotide encoding the variant provided in the present disclosure may be inserted into a chromosome through a vector for chromosomal insertion in a cell. The insertion of a polynucleotide into the chromosome may be performed by any method known in the art, such as homologous recombination, but is not limited thereto. A selection marker for confirming the chromosomal insertion may be further comprised. The selection marker is used to screen cells transformed with the vector, *i.e.*, to confirm the insertion of a desired nucleic acid molecule; markers that confer selectable phenotypes, such as drug resistance, nutrient requirements, resistance to cytotoxic agents, or expression of surface polypeptides, may be used. In an environment treated with a selective agent, only the cells that express the selection marker survive or exhibit a different phenotype, allowing for the selection of transformed cells.

The "host cell" of the present disclosure may be comprised without limitation as long as the host cell is capable of expressing the polypeptide having PET degradation activity of the present disclosure.

The host cell of the present disclosure may comprise the above-described variant, a polynucleotide encoding the variant, a nucleic acid construct and/or vector comprising the same.

In one embodiment, the host cell of the present disclosure may overexpress a polypeptide having PET degradation activity, and the polypeptide having PET degradation activity may be the above-described variant and/or a parent PETase thereof.

The nucleic acid construct or vector may be incorporated into a chromosome as described above, or maintained as a self-replicating extrachromosomal vector.

The host cell of the present disclosure comprises any progeny of a parent cell that is not identical to the parent cell due to mutations occurring during replication.

The host cell can be any cell, for example, a prokaryotic or eukaryotic cell, useful for generating the recombination of the variant.

The prokaryotic host cell may be any Gram-positive or Gram-negative bacterium.

The Gram-positive bacterium comprises *Bacillus*, *Clostridium*, *Enterococcus*, *Geobacillus*, *Lactobacillus*, *Lactococcus*, *Oceanobacillus*, *Staphylococcus*, *Streptococcus, Kutzneria, Corynebacterium,* and *Streptomyces,* but is not limited thereto.

The Gram-negative bacterium comprises *Campylobacter, Escherichia, Flavobacterium, Fusobacterium, Helicobacter, Illiobacter, Neisseria, Pseudomonas, Salmonella, Vibrio* (*e.g., Vibrio natriegens*) and *Ureaplasma,* but is not limited thereto.

In one embodiment, the bacterial host cell may be a host cell of the genus *Bacillus,* and specifically, it comprises *Bacillus alcalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis,* but is not limited thereto.

In one embodiment, the bacterial host cell may be a host cell of the genus *Streptococcus,* and specifically, it comprises *Streptococcus equisimilis, Streptococcus pyogenes, Streptococcus uberis,* and *Streptococcus equi subsp. zooepidemicus,* but is not limited thereto.

In one embodiment, the bacterial host cell may be a host cell of the genus *Streptomyces,* and specifically, it comprises *Streptomyces achromogenes, Streptomyces avermitilis, Streptomyces coelicolor, Streptomyces griseus,* and *Streptomyces lividans,* but is not limited thereto.

In one embodiment, the bacterial host cell may be a host cell of the genus *Corynebacterium,* and may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* but is not limited thereto.

In one embodiment, the bacterial host cell may be a host cell of the genus *Escherichia* and may be *Escherichia coli* (*E. coli*), but is not limited thereto.

The host cell may be a eukaryote, such as a mammal, insect, plant, or fungal cell.

The host cell may be a fungal cell. In the present disclosure, "fungus" comprises Ascomycota, Basidiomycota, Chytridiomycota, and Zygomycota, as well as Oomycota and all imperfect fungi.

Fungal host cells may be yeast cells. In the present disclosure, "yeast" comprises ascosporogenous yeasts (Endomycetales), basidiosporogenous yeasts, and yeasts belonging to the Fungi imperfecti (Blastomycetes). However, this classification may change and may be defined as described in Biology and Activities of Yeast (Skinner, Passmore, and Davenport, editors, Soc. App. Bacteriol. Symposium Series No. 9, 1980).

Yeast host cells may be a *Candida, Hansenula, Kluyveromyces, Pichia, Komagatella, Saccharomyces, Schizosaccharomyces,* or *Yarrowia* cell, for example, a *Kluyveromyces lactis, Saccharomyces carlsbergensis, Saccharomyces cerevisiae, Saccharomyces diastaticus, Saccharomyces douglasii, Saccharomyces kluyveri, Saccharomyces norvensis, Saccharomyces orbensis, Saccharomyces oviformis, Komagatella phaffii,* or *Yarrowia lipolytica* cell.

The fungal host cell may be a filamentous fungal cell. "Filamentous fungi" comprises all filamentous forms of the Mycophylum and Oomyceta subphylum (as defined in the literature (Hawksworth *et al.,* 1995)). Filamentous fungi are generally characterized by hyphae walls consisting of chitin, cellulose, glucan, chitosan, mannan, and other complex polysaccharides. Vegetative growth is by hyphae elongation, and carbon catabolism is absolutely aerobic. In contrast, vegetative growth by yeast, for example, *Saccharomyces cerevisiae,* is by the germination of a unicellular thallus, and carbon catabolism may be fermentable.

Filamentous fungal host cells comprise an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may comprise an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell. However, it is not limited thereto.

The "composition" of the present disclosure may comprise a polypeptide having PET degradation activity, having activity corresponding to that of a polypeptide consisting of the amino acid sequence of SEQ ID NO: 1; and/or a host cell expressing a variant thereof or the polypeptide.

For the polypeptide having PET degradation activity, having activity corresponding to that of a polypeptide consisting of the amino acid sequence of SEQ ID NO: 1, the description of the parent PETase of the variant provided in the present disclosure may be applied. In addition, regarding the variant of the polypeptide and the host cell expressing the same, the description of the variant polypeptide and the host cell expressing the same provided in the present disclosure may be applied.

The composition of the present disclosure can be used to convert polyester into a final product.

The term "polyester" refers to a polymer that contains ester functional groups in the main chain of the structure. For example, polyethylene terephthalate is a semi-aromatic copolymer consisting of two monomers, terephthalic acid, and ethylene glycol.

The polyester may be selected from polyethylene terephthalate (PET), polyethylene terephthalate glycol (PETG), polyethylene co-isosorbide-terephthalate (PEIT), polytrimethylene terephthalate (PTT), polybutylene adipate terephthalate (PBAT), polycyclohexylenedimethylene terephthalate (PCT), and polybutylene terephthalate (PBT). Specifically, the polyester may be PET.

The polypeptide of the present disclosure or a composition comprising the same may be used to depolymerize PET into bis(2-hydroxyethyl) terephthalate (BHET) and/or decompose PET into mono(2-hydroxyethyl) terephthalate (MHET), terephthalic acid (TPA), and ethylene glycol (EG).

In one embodiment, the polypeptide of the present disclosure can decompose polyester by a glycolysis reaction.

For example, the degradation of a polyester may be performed, in the presence of the polypeptide of the present disclosure and ethylene glycol (EG), by comprising converting the polyester to BHET.

For example, the degradation of the polyester may be performed, in the presence of the polypeptide of the present disclosure and ethylene glycol (EG), by comprising reacting the ester group of a polyester and the amino acid residue of the polypeptide to form an acyl-enzyme intermediate; and reacting the formed intermediate with ethylene glycol in a de-acylation step, thereby converting the polyester to BHET.

The composition of the present disclosure may further comprise other components in addition to the polypeptide having PET degradation activity provided in the present disclosure. Those skilled in the art can appropriately select components to be added to the composition of the present disclosure.

In one embodiment, the composition of the present disclosure may further comprise any component suitable for application in converting PET into a final product.

In one embodiment, the composition of the present disclosure may further comprise any component suitable for application in PET degradation.

Examples of substances that may be added comprise stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, *etc*., but are not limited thereto.

In one embodiment, the composition provided in the present disclosure may further comprise a naturally occurring substance or a non-naturally occurring substance in addition to the variant provided in the present disclosure. In one embodiment, the composition provided in the present disclosure may further comprise water.

In one embodiment, the composition provided in the present disclosure may further comprise additional enzymes in addition to the variant provided in the present disclosure.

The method for producing a variant of the present disclosure may comprise: culturing a host cell; and recovering a variant expressed in the culturing.

As used herein, the term "culturing" means that the host cell is grown under appropriately controlled environmental conditions. The culturing process of the present disclosure may be performed in a suitable medium known in the art under suitable culturing conditions known in the art. Such a culturing process may be readily adjusted and used by those skilled in the art according to the selected strain. Specifically, the culturing may be batch culturing, continuous culturing, or fed-batch culturing, but is not limited thereto.

As used herein, the term "medium" refers to a mixed substance containing nutrients required to culture the host cell as a main component, and the medium supplies nutrients, growth factors, *etc*., comprising water, which are indispensable for survival and development. Specifically, any medium and culture conditions may be used for culturing the host cell without particular limitation, as long as the medium is used for the common culture of host cells. The host cell may be cultured in a common medium containing suitable carbon sources, nitrogen sources, phosphorus sources, inorganic compounds, amino acids, and/or vitamins, *etc*., while controlling the temperature, pH, *etc*. under aerobic conditions.

In the present disclosure, the carbon sources comprise carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamate, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e.*, molasses converted into reducing sugars) may be used, and other various carbon sources in appropriate amounts may be used without limitation. These carbon sources may be used alone or in combination of at least two, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamate, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of at least two, but are not limited thereto.

As the phosphorus sources, monobasic potassium phosphate, dibasic potassium phosphate, or the corresponding sodium-containing salts may be used. As for inorganic compounds, sodium chloride, calcium chloride, iron chloride, magnesium sulfate, iron sulfate, manganese sulfate, calcium carbonate, *etc*., may be used, and in addition, amino acids, vitamins, and/or suitable precursors may be comprised. These components or precursors may be added to the medium in a batchwise or continuous manner. However, the medium is not limited thereto.

During the culturing of the host cell, compounds such as ammonium hydroxide, potassium hydroxide, ammonia, phosphoric acid, and sulfuric acid may be added to the medium in an appropriate manner to adjust the pH of the medium. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas or nitrogen, hydrogen, or carbon dioxide gas may be injected. However, the culturing conditions are not limited thereto

The temperature of the medium may be from 20°C to 50°C, specifically from 25°C to 40°C, but is not limited thereto. The culturing may be continued until a desired amount of useful material is obtained, specifically for 24 hours to 196 hours, but is not limited thereto.

In one embodiment, the variant expressed during the culturing can be recovered using a method known in the art to which the present disclosure pertains. For example, the variant can be recovered from the nutrient medium by conventional procedures comprising, but not limited to, collection, centrifugation, filtration, extraction, spray-drying, evaporation, or precipitation.

The method of recovery may be collecting the desired variant using a suitable method known in the art depending on the method for culturing the host cell of the present disclosure, such as batch, continuous, or fed-batch culturing. For example, the variant can be recovered from the medium or the host cell using suitable methods known in the art, such as centrifugation, filtration, crystallization, treatment with protein precipitants (salting-out method), extraction, ultrasonic disruption, ultrafiltration, dialysis, various types of chromatography such as molecular sieve chromatography (gel filtration), adsorption chromatography, ion exchange chromatography, and affinity chromatography, HPLC, and a combination thereof.

In another embodiment, the variant expressed by the host cell during the culturing may not be recovered. In the above embodiment, the host cell expressing the variant can be used as a source of the variant.

The present disclosure may comprise a method for preparing bis-2-hydroxyethyl terephthalate (BHET), (mono(2-hydroxymethyl) terephthalate (MHET), terephthalic acid (TPA), and/or ethylene glycol (EG), comprising contacting polyester with the above-described polypeptide having PET degradation activity, a variant thereof, a host cell expressing the polypeptide, and/or a composition comprising the same.

Specifically, polyester may comprise the above-described description.

In the present disclosure, "contacting" a variant polypeptide, a host cell expressing the polypeptide, or a composition comprising the polypeptide with the polyester means degrading the polyester, but is not limited thereto. Decomposition can also be used for depolymerization.

The time required to decompose the polyester may vary depending on several process parameters (*i.e.*, temperature, pH, additional chemicals, *etc.*), as well as any article comprising the polyester (*i.e.*, properties, origin, composition, shape, *etc*. of the plastic product) and the form and amount of the variant polypeptide used. Process parameters can be easily applied in a manner suitable for articles comprising the polyester using techniques known in the art.

For example, the decomposition process may be carried out at 20°C to 90°C, specifically at 40°C to 80°C, and more specifically at 50°C to 70°C. More specifically, the temperature may be maintained below an inactivation temperature corresponding to the temperature at which the variant polypeptide is inactivated and/or below a temperature at which the host cell no longer synthesizes the variant polypeptide.

For example, the decomposition process may be carried out at pH 5 to pH 11, and specifically at pH 6 to pH 9.

In an embodiment of the present disclosure, an article comprising the polyester may be pretreated before contacting with the variant polypeptide of the present disclosure to physically or chemically modify the structure thereof, thereby increasing the contact area with the variant polypeptide.

Specifically, BHET, MHET, TPA, and/or EG that may be generated from the decomposition by the contact may be recovered sequentially or continuously.

Specifically, the recovered BHET, MHET, TPA, and/or EG can be further purified using any suitable purification method and produced in a re-polymerizable form. More specifically, purification may comprise a stripping process, separation by aqueous solution, steam selective condensation, filtration and concentration of the medium after a bioprocess, separation, distillation, vacuum evaporation, extraction, electrodialysis, absorption, ion exchange, precipitation, crystallization, concentration and acid addition dehydration and precipitation, nanofiltration, acid catalytic treatment, semi-continuous mode distillation or continuous distillation, solvent extraction, evaporative concentration, evaporative crystallization, liquid/liquid extraction, hydrogenation, azeotropic distillation process, absorption, column chromatography, simple vacuum distillation, and microfiltration, but is not limited thereto.

The final products BHET, MHET, TPA, and/or EG obtained using the composition of the present disclosure can be reused in the polymerization of polyester.

Specifically, the final products, BHET, MHET, TPA, and/or EG, can be reused to synthesize polyesters as repolymerizable monomers and/or oligomers. Specifically, polyesters of the same properties can be repolymerized, and by mixing with other monomers and/or oligomers, for example, new copolymers can be synthesized.

The polyester may be selected from polyethylene terephthalate (PET), polyethylene terephthalate glycol (PETG), polyethylene co-isosorbide-terephthalate (PEIT), polytrimethylene terephthalate (PTT), polybutylene adipate terephthalate (PBAT), polycyclohexylenedimethylene terephthalate (PCT), and polybutylene terephthalate (PBT). Specifically, the polyester may be PET.

The method for synthesizing polyester using BHET, MHET, TPA, and/or EG is known in the art.

### Examples

Hereinafter, the present disclosure will be described in more detail by way of Examples and Experimental Examples. However, these Examples and Experimental Examples are given for illustrative purposes only, and the scope of the present disclosure is not intended to be limited by these Examples and Experimental Examples.

### Example 1: Novel PETase discovery

In order to discover novel PETases besides the well-known PET hydrolase (IsPETase) of *Ideonella sakaiensis* 201-F6, sequence homology analysis was performed using the NCBI database to derive 10 PETase candidates. To investigate the 10 selected PETase candidates, attempts were made to produce these enzymes in a signal peptide-cleavage form, and 9 PETase candidates were successfully formed. Then, the PET hydrolysis activity of the 9 PETase candidates was measured by monitoring the amounts of released PET hydrolysis products, MHET, and TPA, using PET bottle powder (PET bottle-derived PET sample; hereinafter referred to as "B-PET") as a substrate. B-PET was obtained through the following processing. Transparent PET bottles were crushed using a crusher, and then the crushed PET was melted in a high-temperature oven at 270°C. The molten PET was immediately immersed in 4°C water and cured. The obtained cured PET underwent a cryogenic grinding process, and then PET powder of 300 µm or less was obtained using a steel mesh. The thus-obtained B-PET was mixed with each PETase candidate and reacted at 50°C for 24 hours. Most PETase candidates showed very small amounts of PET hydrolysis products, whereas WP_116180173.1, an alpha-beta hydrolase of *Kutzneria buriramensis*, showed a significant amount of PET hydrolysis products compared to other enzymes. In addition, to investigate the thermal stability of these enzymes, the melting temperature (Tm) of the nine PETase candidates was measured, and these PETase candidates showed various Tm values ranging from 38.6°C to 87.6°C. WP_116180173.1, which has extremely high PET hydrolysis activity compared to other enzymes, showed high temperature stability with a Tm value of 87.6°C, and WP_116180173.1 showed a relatively high level of soluble expression. These results indicated that WP_116180173.1 possesses excellent characteristics for efficient PET degradation, such as enzymatic activity, thermal stability, and protein expression levels. Accordingly, WP_116180173.1 (PETase of *Kutzneria buriramensis*, hereinafter referred to as "KbPETase"; SEQ ID NO: 1) was selected as the novel PETase in the present disclosure.

### Example 2: Preparation of PET-degraded protein

The expression and purification of KbPETase were performed under the following conditions. The gene of codon-optimized KbPETase (SEQ ID NO: 2) for *E. coli* was synthesized and amplified via polymerase chain reaction (PCR). The nucleotide sequence corresponding to the signal peptide was removed from the synthetic DNA. Afterwards, the PCR product was subcloned (Nde I and Xho I) into pET22b(+) (Novagen) which does not have its own signal peptide. The E. coli Rosetta gami-B (DE3) strain was transformed with the generated expression vector pET22b(+): KbPETase. The *E. coli* strain was cultured at 600 nm with an optical density of 0.6 in a flask containing 1 L of lysogeny broth medium containing 100 mg/L ampicillin at 37°C.

Protein expression was induced by the addition of 0.1 mM isopropyl β-D-1-thiogalactopyranoside (IPTG), and the culture medium was further incubated at 18°C for 18 hours. Next, the cells were harvested by centrifuging at 4000 rpm for 20 minutes at 4°C.

The cell pellet was resuspended in buffer A (40 mM Tris-HCl, pH 8.0) and then pulverized by sonication. Cell debris was removed by centrifugation at 13,500 rpm for 30 minutes, and the supernatant was applied to a Ni-NTA agarose column (Qiagen). After washing with buffer A comprising 30 mM imidazole, the binding protein was eluted with 300 mM imidazole in buffer A. All purification steps were performed at 4°C. The purity of the protein was confirmed by sodium dodecyl sulfate-polyacrylamide gel electrophoresis (SDS-PAGE). The purified protein was concentrated in buffer A. The known PET hydrolysis enzymes LCC (GenBank: AEV21261.1) and IsPETase (GenBank: GAP38373.1) were prepared using the same method and used as a control group. The primers used for cloning are listed in Table 1.

**[Table 1]**

| SEQ ID NO | Enzyme | Primer | Sequence |
|---|---|---|---|
| 66 | KbPETase^{W T} | F | ATATCATATGGCTGACCAAGTGGGACAAGC ACCG |
| 67 | | R | ATATCTCGAGACACGCGGTCGAGCGAAACG C |
| 68 | IsPETase^{WT} | F | TATACATATGCGCGGTCCGAA TCCGACAGCC GCC |
| 69 | | R | GCGCCTCGAGGCTGCAATTCGCTGTACGAA AATC |
| 70 | LCC^{WT} | F | TATATCATATGCAATCCAACCCGTACCAG CGCGG |
| 71 | | R | TATATCTCGAGCTGGCAGTGGCGGTTGTTC GTC |

### Example 3: Analysis of PET degradation activity of KbPETase and known PET-degrading proteins

To compare the PET-hydrolysis activity of KbPETase with that of the widely known PET hydrolysis enzymes LCC (GenBank: AEV21261.1) and IsPETase (GenBank: GAP38373.1), 15 mg of B-PET was prepared by the same method as in Example 1 and immersed in 1 mL of 50 mM glycine-NaOH (pH 9.0) buffer with 500 nM enzyme. The reaction mixture was reacted at 40°C and 50°C for 12 hours. After the reaction, the products of each reaction were analyzed via HPLC to evaluate PET degradation activity.

As the reaction temperature increased, KbPETase showed very high activity relative to IsPETase and LCC, and especially at 50°C, it showed 2.5 times higher activity compared to LCC and 5.5 times higher activity compared to IsPETase. Even at 40°C, KbPETase showed approximately twice the activity compared to these two enzymes. (FIG. 1). These results indicate that KbPETase can have higher PET hydrolysis activity than IsPETase and LCC, and especially can have high PET hydrolysis activity even at high temperatures.

### Example 4: Preparation of KbPETase variants and measurement of thermal stability and PET degradation activity of the variants

To improve the PET degradation activity and thermal stability of the enzyme, 41 KbPETase variants were prepared in the same manner as in Example 2. To compare the activities of wild-type KbPETase and the KbPETase variants, 15 mg of B-PET was prepared and immersed in 1 mL of 50 mM glycine-NaOH (pH 9.0) buffer with 500 nM enzyme. The reaction mixture was reacted at 60°C for 6 to 24 hours. After the reaction, the products of each reaction were analyzed via HPLC to evaluate PET degradation activity. In addition, thermal stability was confirmed by measuring the melting temperature (the temperature at which protein denaturation occurs, Tm) of each variant. The melting temperature was determined by measuring the melting curve using StepOnePlus Real-Time PCR (Thermo Fisher Scientific) with a Protein thermal shift dye (Applied Biosystems). Specifically, 5 µg of KbPETase was mixed with 20 µL of protein thermal shift dye, and the signal alteration indicating the start of protein modification was monitored while varying the temperature from 25°C to 99°C at a rate of 0.1°C per second. Based on the melting curves, the melting temperature (Tm) of KbPETaseWT and 41 variants was determined.

The variant M1 showed approximately 1.24 times higher PET degradation activity compared to KbPETaseWT. The variant M2 showed approximately 1.12 times higher PET degradation activity compared to KbPETaseWT. The variant M3 showed approximately 1.07 times higher PET degradation activity compared to KbPETaseWT. The variant M4 showed approximately 1.36 times higher PET degradation activity compared to KbPETaseWT. The variant M5 showed approximately 1.03 times higher PET degradation activity compared to KbPETaseWT. The variant M6 showed approximately 1.17 times higher PET degradation activity compared to KbPETaseWT. The variant M7 showed approximately 1.14 times higher PET degradation activity compared to KbPETaseWT. The variant M8 showed approximately 1.27 times higher PET degradation activity compared to KbPETaseWT. The variant M9 showed approximately 1.1 times higher PET degradation activity compared to KbPETaseWT. The variant M10 showed approximately 1.09 times higher PET degradation activity compared to KbPETaseWT. The variant M11 showed approximately 1.25 times higher PET degradation activity compared to KbPETaseWT. The variant M12 showed approximately 1.05 times higher PET degradation activity compared to KbPETaseWT. The variant M13 showed approximately 1.2 times higher PET degradation activity compared to KbPETaseWT. The variant M14 showed approximately 1.06 times higher PET degradation activity compared to KbPETaseWT. The variant M15 showed approximately 1.19 times higher PET degradation activity compared to KbPETaseWT. The variant M16 showed approximately 1.23 times higher PET degradation activity compared to KbPETaseWT. The variant M17 showed approximately 1.54 times higher PET degradation activity compared to KbPETaseWT. The variant M18 showed approximately 1.19 times higher PET degradation activity compared to KbPETaseWT. The variant M19 showed approximately 1.55 times higher PET degradation activity compared to KbPETaseWT. The variant M20 showed approximately 1.15 times higher PET degradation activity compared to KbPETaseWT. The variant M21 showed approximately 1.31 times higher PET degradation activity compared to KbPETaseWT. The variant M22 showed approximately 1.43 times higher PET degradation activity compared to KbPETaseWT. The variant M23 showed approximately 1.11 times higher PET degradation activity compared to KbPETaseWT. The variant M24 showed approximately 1.28 times higher PET degradation activity compared to KbPETaseWT. The variant M25 showed approximately 1.23 times higher PET degradation activity compared to KbPETaseWT. The variant M26 showed approximately 1.63 times higher PET degradation activity compared to KbPETaseWT. The variant M27 showed approximately 1.26 times higher PET degradation activity compared to KbPETaseWT. The variant M28 showed approximately 1.57 times higher PET degradation activity compared to KbPETaseWT. The variant M29 showed approximately 1.59 times higher PET degradation activity compared to KbPETaseWT. The variant M30 showed approximately 1.48 times higher PET degradation activity compared to KbPETaseWT. The variant M31 showed approximately 1.67 times higher PET degradation activity compared to KbPETaseWT. The variant M32 showed an increase of 5.8°C in the Tm value and approximately 1.38 times higher PET degradation activity compared to KbPETaseWT. The variant M33 showed an increase of 3.1°C in the Tm value and approximately 1.13 times higher PET degradation activity compared to KbPETaseWT. The variant M34 showed an increase of 11.4°C in the Tm value and approximately 1.45 times higher PET degradation activity compared to KbPETaseWT. The variant M35 showed an increase of 1.6°C in the Tm value and approximately 1.02 times higher PET degradation activity compared to KbPETaseWT. The variant M36 showed an increase of 0.8°C in the Tm value and approximately 1.24 times higher PET degradation activity compared to KbPETaseWT. The M37 variant showed an increase of 4.1°C in the Tm value compared to KbPETaseWT, but the PET degradation activity decreased slightly. The variant M38 showed an increase of 9.2°C in the Tm value and approximately 1.06 times higher PET degradation activity compared to KbPETaseWT. The variant M39 showed an increase of 12.3°C in the Tm value and approximately 3.67 times higher PET degradation activity compared to KbPETaseWT. The variant M40 showed an increase of 12.3°C in the Tm value and approximately 2.37 times higher PET degradation activity compared to KbPETaseWT. The variant M41 showed an increase of 12.3°C in the Tm value and approximately 3.95 times higher PET degradation activity compared to KbPETaseWT.

Based on the above, it was confirmed that introducing mutations into the KbPETase protein increased the temperature stability of the protein or enhanced PET degradation activity.

**[Table 2]**

| Mutant | Product concentration (µM, 12hr) | Tm (°C) |
|---|---|---|
| KbPETaseWT | 1189.60 | 87.6 |
| M1(KbPETaseT95R) | 1472.53 | 86.7 |
| M2(KbPETaseD239S) | 1336.90 | 83.5 |
| M3(KbPETaseA279S) | 1272.62 | 86.3 |
| M4(KbPETaseT119N) | 1616.81 | 86.8 |
| M5(KbPETaseV184I) | 1228.00 | 84.8 |
| M6(KbPETaseT95R/D239S) | 1392.01 | 85.7 |
| M7(KbPETaseT95R/A279S) | 1353.87 | 86.7 |
| M8(KbPETaseT95R/T119N) | 1513.94 | 86.8 |
| M9(KbPETaseT95R/V184I) | 1310.36 | 85.4 |
| M10(KbPETaseD239S/A279S) | 1294.61 | 84.9 |
| M11(KbPETaseD239S/T119N) | 1491.72 | 85.1 |
| M12(KbPETaseD239S/V184I) | 1253.18 | 84.2 |
| M13(KbPETaseA279S/T119N) | 1434.54 | 86.5 |
| M14(KbPETaseA279S/V184I) | 1263.12 | 85.4 |
| M15(KbPETaseT119N/V184I) | 1418.37 | 86.0 |
| M16(KbPETaseT95R/D239S/A279S) | 1461.64 | 85.9 |
| M17(KbPETaseT95R/D239S/T119N) | 1834.09 | 86.3 |
| M18(KbPETaseT95R/D239S/V184I) | 1410.57 | 85.3 |
| M19(KbPETaseT95R/A279S/T119N) | 1837.94 | 86.7 |
| M20(KbPETaseT95R/A279S/V184I) | 1370.48 | 85.6 |
| M21(KbPETaseT95R/T119N/V184I) | 1562.16 | 85.6 |
| M22(KbPETaseD239S/A279S/T119N) | 1706.49 | 85.9 |
| M23(KbPETaseD239S/A279S/V184I) | 1316.72 | 84.9 |
| M24(KbPETaseD239S/T119N/V184I) | 1524.64 | 85.0 |
| M25(KbPETaseA279S/T119N/V184I) | 1467.05 | 85.6 |
| M26(KbPETaseT95R/D239S/A279S/T119N) | 1934.54 | 86.2 |
| M27(KbPETaseT95R/D239S/A279S/V184I) | 1501.27 | 85.5 |
| M28(KbPETaseT95R/D239S/T119N/V184I) | 1873.46 | 85.4 |
| M29(KbPETaseT95R/A279S/T119N/V184I) | 1894.51 | 85.6 |
| M30(KbPETaseD239S/A279S/T119N/V184I) | 1756.97 | 85.3 |
| M31(KbPETaseT95R/D239S/A279S/T119N/V18 4I) | 1986.76 | 85.7 |

**[Table 3]**

| Mutant | Product concentration (µM, 24hr) | Tm (°C) |
|---|---|---|
| KbPETaseWT | 1982.14 | 87.6 |
| M32(KbPETaseA173C/K197C) | 2745.09 | 93.4 |
| M33(KbPETaseA236C/S281C) | 2247.89 | 90.7 |
| M34(KbPETaseA173C/K197C/A236C/S281C) | 2876.67 | >99.9 |

**[Table 4]**

| Mutant | Product concentration (µM, 6hr) | Tm (°C) |
|---|---|---|
| KbPETaseWT | 968.07 | 87.6 |
| M35(KbPETaseE131Q) | 984.93 | 89.2 |
| M36(KbPETaseQ127S/E131Q) | 1197.26 | 88.4 |
| M37(KbPETaseD190H) | 945.02 | 91.7 |
| M38(KbPETaseQ127S/E131Q/D190H) | 1023.48 | 96.8 |
| M39(T95R/D239S/A279S/T119N/V184I/A173C/ K197C/A236C/S281C) | 3567.57 | >99.9 |
| M40(A173C/K197C/A236C/S281C/Q127S/E131 Q/D190H) | 2289.73 | >99.9 |
| M41(T95R/D239S/A279S/T119N/V184I/A173C/ K197C/A236C/S281C/Q127S/E131Q/D190H) | 3819.47 | >99.9 |

### Example 5: Effect on KbPETase variant site

In order to confirm the changes in protein thermal stability and PET degradation activity when mutation sites confirmed in Example 4 were substituted with other amino acids, each variant was prepared in the same manner as in Example 2, and the PET degradation activity and thermal stability were evaluated in the same manner as in Example 4 and are shown in Tables 5 to 9.

**[Table 5]**

| Mutant | Product concentration (µM, 12hr) | Tm (°C) | Mutant | Product concentration (µM, 12hr) | Tm (°C) |
|---|---|---|---|---|---|
| KbPETaseWT | 1189.60 | 87.6 | M69 (KbPETaseD239 N) | 1162.74 | 82.9 |
| M42 (KbPETaseT95A) | 916.72 | 85.4 | M70 (KbPETaseD239 Q) | 992.63 | 82.5 |
| M43 (KbPETaseT95I) | 891.25 | 84.7 | M71 (KbPETaseD239 C) | 794.75 | 81.8 |
| M44(KbPETaseT 95L) | 872.61 | 84.9 | M72 (KbPETaseD239 G) | 1093.46 | 82.2 |
| M45(KbPETaseT 95V) | 904.34 | 85.1 | M73 (KbPETaseD239 P) | 367.11 | 81.1 |
| M46(KbPETaseT 95M) | 909.37 | 85.3 | M74 (KbPETaseD239 R) | 543.16 | 82.7 |
| M47(KbPETaseT 95F) | 784.59 | 84.1 | M75 (KbPETaseD239 H) | 621.83 | 82.9 |
| M48(KbPETaseT 95Y) | 803.14 | 84.8 | M76 (KbPETaseD239 K) | 564.97 | 82.6 |
| M49(KbPETaseT 95W) | 541.00 | 83.9 | M77 (KbPETaseD239 E) | 937.24 | 83.3 |
| M50 (KbPETaseT95S) | 1013.45 | 86.9 | M78 (KbPETaseT119 A) | 963.17 | 84.1 |
| M51 (KbPETaseT95N) | 1146.97 | 87.1 | M79 (KbPETaseT119I ) | 916.09 | 84.4 |
| M52(KbPETaseT 95Q) | 1163.58 | 87.1 | M80 (KbPETaseT119 L) | 918.33 | 84.3 |
| M53(KbPETaseT 95C) | 762.49 | 85.7 | M81 (KbPETaseT119 V) | 934.71 | 84.6 |
| M54(KbPETaseT 95G) | 847.93 | 86.2 | M82 (KbPETaseT119 M) | 846.03 | 83.5 |
| M55(KbPETaseT 95P) | 423.82 | 84.0 | M83 (KbPETaseT119 F) | 416.12 | 83.1 |
| M56 (KbPETaseT95H) | 1210.28 | 86.4 | M84 (KbPETaseT119 Y) | 431.95 | 82.8 |
| M57 (KbPETaseT95K) | 1198.94 | 86.6 | M85 (KbPETaseT119 W) | 201.08 | 82.5 |
| M58 (KbPETaseT95D) | 862.84 | 85.4 | M86 (KbPETaseT119 S) | 1030.64 | 87.2 |
| M59 (KbPETaseT95E) | 837.29 | 85.6 | M87 (KbPETaseT119 Q) | 1531.64 | 86.3 |
| M60 (KbPETaseD239 A) | 1126.27 | 85.4 | M88 (KbPETaseT119 C) | 841.60 | 85.6 |
| M61 (KbPETaseD239I ) | 1065.89 | 85.2 | M89 (KbPETaseT119 G) | 807.13 | 84.9 |
| M62(KbPETaseD 239L) | 954.73 | 85.1 | M90 (KbPETaseT119 P) | 751.48 | 83.9 |
| M63 (KbPETaseD239 V) | 1097.42 | 85.2 | M91 (KbPETaseT119 R) | 1474.15 | 86.4 |
| M64(KbPETaseD 239M) | 731.53 | 85.0 | M92 (KbPETaseT119 H) | 1392.76 | 86.2 |
| M65(KbPETaseD 239F) | 319.86 | 82.2 | M93 (KbPETaseT119 K) | 1423.53 | 86.5 |
| M66(KbPETaseD 239Y) | 276.18 | 82.1 | M94 (KbPETaseT119 D) | 1272.81 | 85.8 |
| M67(KbPETaseD 239W) | 137.64 | 81.2 | M95 (KbPETaseT119 E) | 1211.28 | 85.7 |
| M68 (KbPETaseD239 T) | 1276.18 | 83.1 | | | |

**[Table 6]**

| Mutant | Product concentratio n (µM, 24hr) | Tm (°C) | Mutant | Product concentratio n (µM, 24hr) | Tm (°C) |
|---|---|---|---|---|---|
| KbPETaseWT | 1982.14 | 87.6 | M112 (KbPETaseA173E/K197 E) | 1391.57 | 85.3 |
| M96 (KbPETaseK197A) | 2134.72 | 86.9 | M113 (KbPETaseS281A) | 2049.37 | 87.3 |
| M97 (KbPETaseA173I/K1 97I) | 2005.36 | 86.7 | M114 (KbPETaseA236I/S281I ) | 1872.53 | 86.8 |
| M98 (KbPETaseA173L/K1 97L) | 2275.15 | 86.3 | M115 (KbPETaseA236L/S281 L) | 2023.48 | 86.9 |
| M99 (KbPETaseA173V/K1 97V) | 2193.92 | 87.3 | M116 (KbPETaseA236V/S281 V) | 1943.59 | 87.1 |
| M100 (KbPETaseA173M/K 197M) | 1768.49 | 84.2 | M117 (KbPETaseA236M/S28 1M) | 1416.76 | 85.3 |
| M101 (KbPETaseA173F/K1 97F) | 234.81 | 77.5 | M118 (KbPETaseA236F/S281 F) | 177.23 | 73.4 |
| M102 (KbPETaseA173S/K1 97S) | 1813.44 | 84.4 | M119 (KbPETaseA236S) | 1998.39 | 86.5 |
| M103 (KbPETaseA173T/K1 97T) | 1872.85 | 83.6 | M120 (KbPETaseA236T/S281 T) | 1826.41 | 86.3 |
| M104 (KbPETaseA173N/K1 97N) | 1694.63 | 83.3 | M121 (KbPETaseA236N/S281 N) | 1549.27 | 84.7 |
| M105 (KbPETaseA173Q/K 197Q) | 1537.45 | 82.7 | M122 (KbPETaseA236Q/S281 Q) | 1374.85 | 84.4 |
| M106 (KbPETaseA173G/K 197G) | 1714.82 | 87.4 | M123 (KbPETaseA236G/S281 G) | 1868.13 | 87.1 |
| M107 (KbPETaseA173P/K1 97P) | 556.19 | 71.5 | M124 (KbPETaseA236R/S281 R) | 1023.52 | 81.4 |
| M108 (KbPETaseA173R/K1 97R) | 894.28 | 83.9 | M125 (KbPETaseA236H/S281 H) | 437.76 | 80.8 |
| M109 (KbPETaseA173H/K1 97H) | 634.79 | 81.2 | M126 (KbPETaseA236K/S281 K) | 735.88 | 83.6 |
| M110(KbPETaseA17 3K) | 1439.07 | 86.5 | M127 (KbPETaseA236D/S281 D) | 1641.35 | 85.7 |
| M111(KbPETaseA17 3D/K197D) | 1625.42 | 85.8 | M128 (KbPETaseA236E/S281 E) | 1596.73 | 85.2 |

**[Table 7]**

| Mutant | Product concentration (µM, 6hr) | Tm (°C) | Mutant | Product concentration (µM, 6hr) | Tm (°C) |
|---|---|---|---|---|---|
| KbPETaseWT | 968.07 | 87.6 | M147 (KbPETaseD190A) | 671.48 | 81.9 |
| M129 (KbPETaseE131A) | 623.43 | 85.1 | M148 (KbPETaseD190I) | 642.15 | 82.3 |
| M130(KbPETaseE 131I) | 668.28 | 85.6 | M149 (KbPETaseD190L) | 702.59 | 83.1 |
| M131(KbPETaseE 131L) | 654.16 | 85.3 | M150 (KbPETaseD190V) | 623.74 | 82.5 |
| M132(KbPETaseE 131V) | 642.71 | 85.3 | M151 (KbPETaseD190M) | 572.16 | 82.0 |
| M133(KbPETase1 31M) | 598.36 | 84.9 | M152 (KbPETaseD190F) | 713.51 | 83.8 |
| M134(KbPETaseE 131F) | 801.57 | 86.1 | M153 (KbPETaseD190Y) | 756.94 | 84.2 |
| M135(KbPETaseE 131Y) | 814.15 | 86.7 | M154 (KbPETaseD190W) | 361.29 | 81.3 |
| M136(KbPETaseE 131W) | 337.13 | 79.3 | M155 (KbPETaseD190S) | 754.18 | 84.5 |
| M137(KbPETaseE 131S) | 712.66 | 86.0 | M156 (KbPETaseD190T) | 772.36 | 84.1 |
| M138(KbPETaseE 131T) | 745.27 | 86.3 | M157 (KbPETaseD190N) | 816.83 | 87.2 |
| M139 (KbPETaseE131N) | 963.49 | 88.7 | M158 (KbPETaseD190Q) | 827.16 | 87.5 |
| M140 (KbPETaseE131C) | 798.36 | 86.4 | M159 (KbPETaseD190C) | 721.57 | 85.4 |
| M141 (KbPETaseE131G ) | 648.12 | 85.3 | M160 (KbPETaseD190G) | 634.39 | 84.7 |
| M142(KbPETaseE 131P) | 275.49 | 84.2 | M161 (KbPETaseD190P) | 518.45 | 83.2 |
| M143 (KbPETaseE131R) | 876.67 | 86.8 | M162 (KbPETaseD190R) | 907.40 | 90.3 |
| M144 (KbPETaseE131H) | 732.18 | 85.9 | M163 (KbPETaseD190K) | 884.37 | 89.9 |
| M145 (KbPETaseE131K) | 754.69 | 86.9 | M164 (KbPETaseD190E) | 859.56 | 87.8 |
| M146 (KbPETaseE131D) | 921.74 | 87.2 | | | |

**[Table 8]**

| Mutant | Product concentration (µM, 6hr) | Tm (°C) |
|---|---|---|
| KbPETaseWT | 968.07 | 87.6 |
| M165 (KbPETaseQ127S/E131A) | 472.48 | 85.1 |
| M166 (KbPETaseQ127S/E131I) | 504.27 | 85.5 |
| M167 (KbPETaseQ127S/E131L) | 511.29 | 85.3 |
| M168 (KbPETaseQ127S/E131V) | 498.13 | 85.2 |
| M169 (KbPETaseQ127S/E131M) | 440.96 | 84.9 |
| M170 (KbPETaseQ127S/E131F) | 554.31 | 85.7 |
| M171 (KbPETaseQ127S/E131Y) | 582.74 | 85.8 |
| M172 (KbPETaseQ127S/E131W) | 189.26 | 82.4 |
| M173 (KbPETaseQ127S/E131S) | 537.87 | 85.9 |
| M174 (KbPETaseQ127S/E131T) | 569.41 | 86.0 |
| M175 (KbPETaseQ127S/E131N) | 1007.63 | 88.1 |
| M176 (KbPETaseQ127S/E131C) | 934.15 | 87.8 |
| M177 (KbPETaseQ127S/E131G) | 542.76 | 85.1 |
| M178 (KbPETaseQ127S/E131P) | 97.15 | 79.2 |
| M179 (KbPETaseQ127S/E131R) | 726.41 | 86.8 |
| M180 (KbPETaseQ127S/E131H) | 684.72 | 86.7 |
| M181 (KbPETaseQ127S/E131K) | 712.98 | 86.7 |
| M182 (KbPETaseQ127S/E131D) | 607.59 | 86.2 |
| M183 (KbPETaseQ127S) | 623.78 | 86.4 |

**[Table 9]**

| Mutant | Product concentratio n (µM, 12hr) | Tm (°C) |
|---|---|---|
| KbPETaseWT | 1189.60 | 87.6 |
| M184 (KbPETaseA279I) | 1043.19 | 86.1 |
| M185 (KbPETaseA279L) | 1064.67 | 86.3 |
| M186 (KbPETaseA279V) | 1137.43 | 86.4 |
| M187 (KbPETaseA279M) | 834.76 | 85.9 |
| M188 (KbPETaseA279F) | 729.54 | 83.7 |
| M189 (KbPETaseA279Y) | 682.17 | 83.4 |
| M190 (KbPETaseA279W) | 418.93 | 82.9 |
| M191 (KbPETaseA279T) | 1136.81 | 86.1 |
| M192 (KbPETaseA279N) | 943.84 | 85.8 |
| M193 (KbPETaseA279Q) | 907.11 | 85.6 |
| M194 (KbPETaseA279C) | 889.37 | 85.1 |
| M195 (KbPETaseA279G) | 1016.55 | 84.9 |
| M196 (KbPETaseA279P) | 784.91 | 83.7 |
| M197 (KbPETaseA279R) | 861.14 | 84.8 |
| M198 (KbPETaseA279H) | 612.73 | 84.7 |
| M199 (KbPETaseA279K) | 594.67 | 85.0 |
| M200 (KbPETaseA279D) | 854.79 | 86.1 |
| M201 (KbPETaseA279E) | 826.81 | 85.9 |
| M202 (KbPETaseV184A) | 1026.43 | 81.6 |
| M203 (KbPETaseV184L) | 1074.08 | 83.7 |
| M204 (KbPETaseV184M) | 831.59 | 81.6 |
| M205 (KbPETaseV184F) | 134.45 | 68.1 |
| M206 (KbPETaseV184Y) | 92.16 | 66.9 |
| M207 (KbPETaseV184S) | 752.49 | 80.3 |
| M208 (KbPETaseV184T) | 768.23 | 80.7 |
| M209 (KbPETaseV184N) | 483.70 | 72.4 |
| M210 (KbPETaseV184Q) | 451.63 | 71.3 |
| M211 (KbPETaseV184C) | 560.11 | 84.5 |
| M212 (KbPETaseV184G) | 572.82 | 81.8 |
| M213 (KbPETaseV184P) | 112.57 | 64.7 |
| M214 (KbPETaseV184R) | 264.31 | 73.6 |
| M215 (KbPETaseV184H) | 334.84 | 79.9 |
| M216 (KbPETaseV184K) | 297.13 | 76.1 |
| M217 (KbPETaseV184D) | 350.49 | 76.3 |
| M218 (KbPETaseV184E) | 325.66 | 76.1 |

From the results, it was confirmed that when 12 mutation sites of KbPETase were altered to other amino acids, substitution with some amino acids affected the improvement of protein activity or thermal stability. This means that the evaluated amino acid positions have a direct or indirect effect on the activity or thermal stability of the protein.

### Example 6: Measurement of PET depolymerization activity by glycolysis-catalyzed reaction of KbPETase variant

With respect to the KbPETase variant M41 with improved durability, the resistance to EG (ethylene glycol) and the PET depolymerization activity were evaluated via a glycolysis catalytic reaction. The KbPETase variant M41 was prepared in the same manner as in Example 2 to prepare an enzyme-EG mixture comprising 5 µL of an enzyme solution at a concentration of 10.2 mg/mL, 45 µL of buffer A, and 950 µL of a 99.5% EG solution. To evaluate resistance to EG, the enzyme-EG reaction mixture was exposed for 0, 2, 4, 6, 8, and 10 days under conditions of 40°C, 50°C, 60°C, and 70°C. 50 mg of B-PET powder was immersed in each of the treated enzyme-EG reaction mixture solutions. The reaction mixture solution was reacted at 40°C for 24 hours. After the reaction, the product of each reaction solution was analyzed via HPLC to measure the PET depolymerization activity by glycolysis catalytic reaction. The concentrations of the major products (TPA, BHET, and MHET) for the enzymatic PET glycolysis depolymerization reaction are presented in Table 10. As a result of the reaction under these conditions, BHET was produced up to approximately 98% of the major reaction products. In particular, the reaction at 40°C for 24 hours resulted in a BHET concentration of 5.26 mM.

**[Table 10]**

| PET glycolysis depolymerization reaction product of MpPETase variant M41 | | | | |
|---|---|---|---|---|
| EG Exposure temperature (°C) | EG Exposure time (days) | TPA (mM) | MHET (mM) | BHET (mM) |
| 40 | 0 | 0.002296 | 0.090819 | 5.260631 |
| 40 | 2 | 0.001836 | 0.081129 | 5.017862 |
| 40 | 4 | 0.001627 | 0.080527 | 4.755063 |
| 40 | 6 | 0.001678 | 0.064918 | 4.52867 |
| 40 | 8 | 0.001737 | 0.091116 | 5.08801 |
| 40 | 10 | 0.001159 | 0.060663 | 4.742384 |
| 50 | 0 | 0.002296 | 0.090819 | 5.260631 |
| 50 | 2 | 0.001647 | 0.084219 | 5.125902 |
| 50 | 4 | 0.00118 | 0.084847 | 4.902993 |
| 50 | 6 | 0.002327 | 0.065049 | 4.155793 |
| 50 | 8 | 0.002026 | 0.069499 | 4.067525 |
| 50 | 10 | 0.001256 | 0.057763 | 4.242881 |
| 60 | 0 | 0.002296 | 0.090819 | 5.260631 |
| 60 | 2 | 0.001499 | 0.05425 | 3.745311 |
| 60 | 4 | 0.001287 | 0.05225 | 3.539175 |
| 60 | 6 | 0.001449 | 0.041095 | 2.703422 |
| 60 | 8 | 0.001176 | 0.053077 | 2.918284 |
| 60 | 10 | 0.00186 | 0.007245 | 0.433589 |
| 70 | 0 | 0.002296 | 0.090819 | 5.260631 |
| 70 | 2 | 0.000506 | 0.001753 | 0.102696 |
| 70 | 4 | 0.000985 | 0.000379 | 0.008072 |
| 70 | 6 | 0.001142 | 6.6E-05 | 0.000575 |
| 70 | 8 | 0.00042 | 6.31E-05 | 0.000474 |
| 70 | 10 | 0.000616 | 0 | 0 |

As set forth above, those skilled in the art will be able to understand that the present disclosure may be embodied in other specific forms without departing from the technical spirit or essential characteristics thereof. Therefore, the embodiments described above should be construed as being exemplified and not limiting the present disclosure. The scope of the present disclosure should be construed as comprising the meaning and scope of the appended claims rather than the detailed description, and all changes or variations derived from the equivalent concepts fall within the scope of the present disclosure.

### [Sequence Listing]

**[Table 11]**

| # | name | Sequence |
|---|---|---|
| 1 | GenBank: WP_116180173 .1KbPETaseWT (AA sequence) | MQPQQKARRWFTRSSAVLAVVLALAAGGLAATAAPAYADQV GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 2 | KbPETaseWT( Nt sequence) | ATGCAGCCACAGCAGAAGGCACGGCGGTGGTTCACCCGA TCGTCGGCGGTGCTGGCGGTCGTCCTGGCGCTGGCGGCC GGCGGCCTGGCCGCCACCGCCGCACCCGCCTACGCCGAC CAGGTCGGCCAGGCCCCCACCGCGGCGAACATCACCGGT GACGGCAGCTTCGCCACGGCGTCCGCCCCGATCACCAAC CAGACCGGGTTCGGCGGCGGCACCGTCTACTACCCGACG GCGGCCGGCACGTATCCGGTGGTCGCCGTCGTTCCCGGA TTCGTGTCCACCTGGAGCCAGATCAGCTGGCTCGGCCCGC GGGTGGCGTCCTGGGGCTTCGTCGTCGTGGGCGCCGACA CCACCAGCGGGTTCGACAGCCCGTCGCAGCGCGCCGACG AGTTGCTCGCGGCGTTGAACTGGGCGGTGAACTCCGCGC CGGCGGCCGTGCGGGGCAAGGTCGACGGCACGCGCCGG GGCGTGGCCGGCTGGTCCATGGGCGGCGGCGGACCCT TGAGGCGCTGGCCAAGGACACGACCGGCACGGTGAAGGC CGGGGTGCCACTGGCGCCGTGGGACATCGGGCAGGACTT CAGCAAGGTCACCAAGCCGGTGTTCATCGTGGGGGCGCA GAACGACACCATCGCGCCGCCCGCCCAGCACGCCGTCCC GTTCTACAACGCGGCTGCCGGACCGAAGTCCTATCTGGAA CTCGCCGGCGCCGACCACTTCTTCCCGACCACCGCCAACC CCACGGTGTCCAGGGCGATGGTGTCCTGGCTGAAGCGCT TCGTCAGCTCCGACGACCGGTTCACTCCGTTCACCTGCGG GTTCGCCGGCGCGGCGGTGTCGGCCTTCCGGAGCACGGC CTGCTAG |
| 3 | KbPETaseM1 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 4 | KbPETaseM2 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGASHFFPTTANP TVSRAMVSWLKRFVSSDDRFTPFTCGFAGAAVSAFRSTAC |
| 5 | KbPETaseM3 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGASVSAFRSTAC |
| 6 | KbPETaseM4 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTNSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 7 | KbPETaseM5 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGADHFFPTTANPT VSRAMVSWLKRFVSSDDRFTPFTCGFAGAAVSAFRSTAC |
| 8 | KbPETaseM6 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGASHFFPTTANP TVSRAMVSWLKRFVSSDDRFTPFTCGFAGAAVSAFRSTAC |
| 9 | KbPETaseM7 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGASVSAFRSTAC |
| 1 0 | KbPETaseM8 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 1 1 | KbPETaseM9 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTTSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGADHFFPTTANPT VSRAMVSWLKRFVSSDDRFTPFTCGFAGAAVSAFRSTAC |
| 1 2 | KbPETaseM10 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGASHFFPTTANP TVSRAMVSWLKRFVSSDDRFTPFTCGFAGASVSAFRSTAC |
| 1 3 | KbPETaseM11 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTNSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGASHFFPTTANP TVSRAMVSWLKRFVSSDDRFTPFTCGFAGAAVSAFRSTAC |
| 1 4 | KbPETaseM12 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGASHFFPTTANPT VSRAMVSWLKRFVSSDDRFTPFTCGFAGAAVSAFRSTAC |
| 1 5 | KbPETaseM13 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTNSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGASVSAFRSTAC |
| 1 6 | KbPETaseM14 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGADHFFPTTANPT VSRAMVSWLKRFVSSDDRFTPFTCGFAGASVSAFRSTAC |
| 1 7 | KbPETaseM15 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTNSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGADHFFPTTANPT VSRAMVSWLKRFVSSDDRFTPFTCGFAGAAVSAFRSTAC |
| 1 8 | KbPETaseM16 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGASHFFPTTANP TVSRAMVSWLKRFVSSDDRFTPFTCGFAGASVSAFRSTAC |
| 1 9 | KbPETaseM17 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGASHFFPTTANP TVSRAMVSWLKRFVSSDDRFTPFTCGFAGAAVSAFRSTAC |
| 2 0 | KbPETaseM18 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTTSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGASHFFPTTANPT VSRAMVSWLKRFVSSDDRFTPFTCGFAGAAVSAFRSTAC |
| 2 1 | KbPETaseM19 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGASVSAFRSTAC |
| 2 2 | KbPETaseM20 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTTSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGADHFFPTTANPT VSRAMVSWLKRFVSSDDRFTPFTCGFAGASVSAFRSTAC |
| 2 3 | KbPETaseM21 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGADHFFPTTANPT VSRAMVSWLKRFVSSDDRFTPFTCGFAGAAVSAFRSTAC |
| 2 4 | KbPETaseM22 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTNSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGASHFFPTTANP TVSRAMVSWLKRFVSSDDRFTPFTCGFAGASVSAFRSTAC |
| 2 5 | KbPETaseM23 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGASHFFPTTANPT VSRAMVSWLKRFVSSDDRFTPFTCGFAGASVSAFRSTAC |
| 2 6 | KbPETaseM24 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTNSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGASHFFPTTANPT VSRAMVSWLKRFVSSDDRFTPFTCGFAGAAVSAFRSTAC |
| 2 | KbPETaseM25 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTNSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGADHFFPTTANPT VSRAMVSWLKRFVSSDDRFTPFTCGFAGASVSAFRSTAC |
| 2 8 | KbPETaseM26 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGASHFFPTTANP TVSRAMVSWLKRFVSSDDRFTPFTCGFAGASVSAFRSTAC |
| 2 9 | KbPETaseM27 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTTSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGASHFFPTTANPT VSRAMVSWLKRFVSSDDRFTPFTCGFAGASVSAFRSTAC |
| 3 0 | KbPETaseM28 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGASHFFPTTANPT VSRAMVSWLKRFVSSDDRFTPFTCGFAGAAVSAFRSTAC |
| 3 1 | KbPETaseM29 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGADHFFPTTANPT VSRAMVSWLKRFVSSDDRFTPFTCGFAGASVSAFRSTAC |
| 3 2 | KbPETaseM30 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTNSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGASHFFPTTANPT VSRAMVSWLKRFVSSDDRFTPFTCGFAGASVSAFRSTAC |
| 3 3 | KbPETaseM31 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSQRADELLAALNWAVNSAPAAVRGKVDGTRRGVAGWSMG GGGTLEALAKDTTGTVKAGIPLAPWDIGQDFSKVTKPVFIVGA QNDTIAPPAQHAVPFYNAAAGPKSYLELAGASHFFPTTANPT VSRAMVSWLKRFVSSDDRFTPFTCGFAGASVSAFRSTAC |
| 3 4 | KbPETaseM32 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALCKDTTGTVKAGVPLAPWDIGQDFSCVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 3 5 | KbPETaseM33 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELCGADHFFPTTANP TVSRAMVSWLKRFVSSDDRFTPFTCGFAGAAVCAFRSTAC |
| 3 6 | KbPETaseM34 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALCKDTTGTVKAGVPLAPWDIGQDFSCVTKPVFIVG AQNDTIAPPAQHAVPFYNAAAGPKSYLELCGADHFFPTTANP TVSRAMVSWLKRFVSSDDRFTPFTCGFAGAAVCAFRSTAC |
| 3 7 | KbPETaseM35 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADQLLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 3 8 | KbPETaseM36 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSSRADQLLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 3 9 | KbPETaseM37 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWHIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 4 0 | KbPETaseM38 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSSRADQLLAALNWAVNSAPAAVRGKVDGTRRGVAGWSMG GGGTLEALAKDTTGTVKAGVPLAPWHIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 4 1 | KbPETaseM39 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSQRADELLAALNWAVNSAPAAVRGKVDGTRRGVAGWSMG GGGTLEALCKDTTGTVKAGIPLAPWDIGQDFSCVTKPVFIVGA QNDTIAPPAQHAVPFYNAAAGPKSYLELCGASHFFPTTANPT VSRAMVSWLKRFVSSDDRFTPFTCGFAGASVCAFRSTAC |
| 4 2 | KbPETaseM40 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSSRADQLLAALNWAVNSAPAAVRGKVDGTRRGVAGWSMG GGGTLEALCKDTTGTVKAGVPLAPWHIGQDFSCVTKPVFIVG AQNDTIAPPAQHAVPFYNAAAGPKSYLELCGADHFFPTTANP TVSRAMVSWLKRFVSSDDRFTPFTCGFAGAAVCAFRSTAC |
| 4 3 | KbPETaseM41 | MQPQQKARRWFTRSSAVLAVVLALAAGGLAATAAPAYADQV GQAPTAANITGDGSFATASAPITNQTGFGGGTVYYPTAAGTY PVVAVVPGFVSRWSQISWLGPRVASWGFVVVGADTNSGFDS PSSRADQLLAALNWAVNSAPAAVRGKVDGTRRGVAGWSMG GGGTLEALCKDTTGTVKAGIPLAPWHIGQDFSCVTKPVFIVGA QNDTIAPPAQHAVPFYNAAAGPKSYLELCGASHFFPTTANPT VSRAMVSWLKRFVSSDDRFTPFTCGFAGASVCAFRSTAC |
| 4 4 | KbPETaseM56 | MQPQQKARRWFTRSSAVLAVVLALAAGGLAATAAPAYADQV GQAPTAANITGDGSFATASAPITNQTGFGGGTVYYPTAAGTY PVVAVVPGFVSHWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 4 5 | KbPETaseM57 | MQPQQKARRWFTRSSAVLAVVLALAAGGLAATAAPAYADQV GQAPTAANITGDGSFATASAPITNQTGFGGGTVYYPTAAGTY PVVAVVPGFVSKWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 4 6 | KbPETaseM68 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGATHFFPTTANPT VSRAMVSWLKRFVSSDDRFTPFTCGFAGAAVSAFRSTAC |
| 4 7 | KbPETaseM87 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTQSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 4 8 | KbPETaseM91 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTRSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 4 9 | KbPETaseM92 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTHSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 5 0 | KbPETaseM93 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTKSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 5 1 | KbPETaseM94 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTDSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 5 2 | KbPETaseM95 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTESGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 5 3 | KbPETaseM96 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSAVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 5 4 | KbPETaseM97 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALIKDTTGTVKAGVPLAPWDIGQDFSIVTKPVFIVGA QNDTIAPPAQHAVPFYNAAAGPKSYLELAGADHFFPTTANPT VSRAMVSWLKRFVSSDDRFTPFTCGFAGAAVSAFRSTAC |
| 5 5 | KbPETaseM98 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALLKDTTGTVKAGVPLAPWDIGQDFSLVTKPVFIVGA QNDTIAPPAQHAVPFYNAAAGPKSYLELAGADHFFPTTANPT VSRAMVSWLKRFVSSDDRFTPFTCGFAGAAVSAFRSTAC |
| 5 6 | KbPETaseM99 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALVKDTTGTVKAGVPLAPWDIGQDFSVVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 5 7 | KbPETaseM113 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVAAFRSTAC |
| 5 8 | KbPETaseM115 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELLGADHFFPTTANPT VSRAMVSWLKRFVSSDDRFTPFTCGFAGAAVLAFRSTAC |
| 5 9 | KbPETaseM119 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELSGADHFFPTTANP TVSRAMVSWLKRFVSSDDRFTPFTCGFAGAAVSAFRSTAC |
| 6 0 | KbPETaseM139 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADNLLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 6 1 | KbPETaseM162 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWRIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 6 2 | KbPETaseM163 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWKIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 6 3 | KbPETaseM164 | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSQRADELLAALNWAVNSAPAAVRGKVDGTRRGVAGWSMG GGGTLEALAKDTTGTVKAGVPLAPWEIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 6 4 | KbPETase M175 (Q127S/E131N) | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSSRADNLLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 6 5 | KbPETase M176 (Q127S/E131C) | MQPQQKARRWFTRSSAVLAVVLALAAGGLAATAAPAYADQV GQAPTAANITGDGSFATASAPITNQTGFGGGTVYYPTAAGTY PVVAVVPGFVSTWSQISWLGPRVASWGFVVVGADTTSGFDS PSSRADCLLAALNWAVNSAPAAVRGKVDGTRRGVAGWSMG GGGTLEALAKDTTGTVKAGVPLAPWDIGQDFSKVTKPVFIVG AQNDTIAPPAQHAVPFYNAAAGPKSYLELAGADHFFPTTANP TVSRAMVSWLKRFVSSDDRFTPFTCGFAGAAVSAFRSTAC |
| 6 6 | | ATATCATATGGCTGACCAAGTGGGACAAGCACCG |
| 6 7 | | ATATCTCGAGACACGCGGTCGAGCGAAACGC |
| 6 8 | | TATACATATGCGCGGTCCGAA TCCGACAGCC GCC |
| 6 9 | | GCGCCTCGAGGCTGCAATTCGCTGTACGAAAATC |
| 7 0 | | TATATCATATGCAATCCAACCCGTACCAG CGCGG |
| 7 1 | | TATATCTCGAGCTGGCAGTGGCGGTTGTTCGTC |

## Claims

1. A variant polypeptide having PET ( polyethylene terephthalate) degradation activity, wherein:
i) the variant polypeptide has sequence identity of at least 70% and less than 100% to SEQ ID NO: 1; and/or
ii) the variant polypeptide corresponds to a polypeptide encoded by a polynucleotide having sequence identity of at least 70% and less than 100% to a sequence encoding a mature polypeptide of SEQ ID NO: 1; and/or
iii) the variant polypeptide corresponds to a polypeptide encoded by (a) a mature polypeptide coding sequence of SEQ ID NO: 1, (b) a cDNA thereof, or (c) a polynucleotide that hybridizes to the full-length complement of (a) or (b) under low stringency conditions, medium stringency conditions, medium-high stringency conditions, high stringency conditions, or very high stringency conditions; and/or
iv) the variant polypeptide corresponds to a functional fragment of a polypeptide of i), ii), or iii) having PET degradation activity, and
wherein the variant polypeptide comprises any one modification selected from the following at one or more positions selected from positions 95, 119, 184, 239, 279, 173, 197, 236, 281, 127, 131, and 190:
amino acid deletion, amino acid insertion, substitution with another amino acid, disulfide bond formation, and/or a combination thereof,
wherein the position numbers correspond to the positions in the polypeptide of SEQ ID NO: 1.

2. The variant polypeptide of claim 1, wherein, in the variant polypeptide having PET degradation activity prior to modification, the amino acid at position 95 is threonine (T); the amino acid at position 119 is threonine (T); the amino acid at position 184 is valine (V); the amino acid at position 239 is aspartate (D); the amino acid at position 279 is alanine (A); the amino acid at position 173 is alanine (A); the amino acid at position 197 is lysine (K); the amino acid at position 236 is alanine (A); the amino acid at position 281 is serine (S); the amino acid at position 127 is glutamine (Q); the amino acid at position 131 is glutamate (E); and/or the amino acid at position 190 is aspartate (D).

3. The variant polypeptide of claim 1, wherein the variant polypeptide comprises one or more of the following substitutions:
substitution of amino acid corresponding to position 95 with arginine, histidine, or lysine;
substitution of amino acid corresponding to position 119 with asparagine, glutamine, arginine, histidine, lysine, aspartate, or glutamate;
substitution of amino acid corresponding to position 184 with isoleucine;
substitution of amino acid corresponding to position 239 with serine or threonine;
substitution of amino acid corresponding to position 279 with serine;
substitution of amino acid corresponding to position 173 with cysteine, isoleucine, leucine, or valine;
substitution of amino acid corresponding to position 197 with cysteine, alanine, isoleucine, leucine, or valine;
substitution of amino acid corresponding to position 236 with cysteine, leucine, or serine;
substitution of amino acid corresponding to position 281 with cysteine, alanine, or leucine;
substitution of amino acid corresponding to position 127 with serine;
substitution of amino acid corresponding to position 131 with glutamine, asparagine, or cysteine; and
substitution of amino acid corresponding to position 190 with histidine, arginine, lysine, or glutamate,
wherein the position numbers correspond to the positions in the polypeptide of SEQ ID NO: 1.

4. The variant polypeptide of claim 3, wherein the variant polypeptide comprises one or more substitutions selected from:
one or more substitutions selected from T95R, D239S, A279S, T119N, and V184I;
A173C/K197C;
A236C/S281C; and
one or more substitutions selected from Q127S, E131Q, and D190H,
wherein the position numbers correspond to the positions in the polypeptide of SEQ ID NO: 1.

5. The variant polypeptide of claim 3, wherein the variant polypeptide corresponds to one in which the amino acid at position 95 is substituted with arginine; amino acid at position 239 is substituted with serine; amino acid at position 279 is substituted with serine; amino acid at position 119 is substituted with asparagine; amino acid at position 184 is substituted with isoleucine; amino acid at position 173 is substituted with cysteine; amino acid at position 197 is substituted with cysteine; amino acid at position 236 is substituted with cysteine; amino acid at position 281 is substituted with cysteine; amino acid at position 127 is substituted with serine; amino acid at position 131 is substituted with glutamine; and amino acid at position 190 is substituted with histidine,
wherein the position numbers correspond to the positions in the polypeptide of SEQ ID NO: 1.

6. The variant polypeptide of claim 3, wherein the variant polypeptide forms a disulfide bond between a cysteine pair selected from the following:
cysteine at position 173 and cysteine at position 197; and/or
cysteine at position 236 and cysteine at position 281,
wherein the position numbers correspond to the positions in the polypeptide of SEQ ID NO: 1.

7. The variant polypeptide of claim 1, wherein the variant polypeptide has one or more of modified properties the following i) to vii) relative to a polypeptide consisting of the amino acid sequence of SEQ ID NO: 1:
i) increase or decrease in enzymatic activity;
ii) increase or decrease in a specific activity;
iii) increase or decrease in pH stability;
iv) increase or decrease in storage stability;
v) increase or decrease in acid tolerance;
vi) increase or decrease in thermal tolerance or thermal stability; and
vii) alteration of substrate specificity.

8. A composition comprising the variant polypeptide of any one of claims 1 to 7.

9. Use of the variant polypeptide of any one of claims 1 to 7, a polypeptide of SEQ ID NO: 1 or having at least 70% sequence identity thereto;
a host cell expressing the polypeptide; -
or a composition comprising the polypeptide, for degrading polyester.

10. A polynucleotide encoding the variant polypeptide of any one of claims 1 to 7.

11. A host cell comprising: the variant polypeptide of any one of claims 1 to 7; a polynucleotide encoding the variant polypeptide; a nucleic acid construct comprising the polynucleotide; and/or a vector comprising the nucleotide or the nucleic acid construct.

12. A host cell over-expressing the variant polypeptide of any one of claims 1 to 7, a polypeptide of SEQ ID NO: 1 or having at least 70% sequence identity thereto.

13. A method for preparing a variant polypeptide having PET degradation activity, comprising:
culturing a host cell comprising the variant polypeptide of any one of claims 1 to 7; a polynucleotide encoding the variant polypeptide; a nucleic acid construct comprising the polynucleotide; and/or a vector comprising the nucleotide or the nucleic acid construct; and
recovering a variant polypeptide having PET degradation activity expressed during the culturing.

14. A method for degrading polyester, comprising treating polyester with the variant polypeptide of any one of claims 1 to 7, a polypeptide of SEQ ID NO: 1 or having at least 70% sequence identity thereto;
a host cell expressing the polypeptide; and/or
a composition comprising the polypeptide.

15. The method of claim 14, wherein the polyester is PET.

16. The method of claim 14, wherein the method comprises performing a glycolysis reaction by the polypeptide.

17. The method of claim 14, wherein the method comprises converting the polyester to BHET in the presence of the polypeptide and ethylene glycol.

18. A method for preparing bis-2-hydroxyethyl terephthalate (BHET), (mono(2-hydroxymethyl) terephthalate (MHET), terephthalic acid (TPA), and/or ethylene glycol (EG), comprising contacting polyester with the variant polypeptide of any one of claims 1 to 7, a polypeptide of SEQ ID NO: 1 or having at least 70% sequence identity thereto;
a host cell expressing the polypeptide; and/or
a composition comprising the polypeptide.

19. The method of claim 18, wherein the method further comprises recovering the prepared BHET, MHET, TPA, and/or EG.

20. A method for producing polyester, comprising synthesizing a polyester using BHET, MHET, TPA, and/or EG prepared according to claim 19.

21. The method of claim 20, wherein the polyester is PET.
